# EUROPEAN PATENT APPLICATION

(11) **EP 4 847 630 A2**
(43) Date of publication of application: **02.12.2026**
(21) Application number: 26197727.6
(22) Date of filing: 13.07.2018
(51) Int. Cl.: C12Q 1/6883

(54) **DETECTING TISSUE-SPECIFIC DNA**

(30) Priority: 13.07.2017 US 201762531990 P
(62) Document number(s) of the earlier application(s) in accordance with Art. 76 EPC: 18755924.0 / 3 652 342
(71) Applicant: Yissum Research and Development Company of the Hebrew University of Jerusalem Ltd., 9139002 Jerusalem (IL); Hadasit Medical Research Services and Development Ltd., 9112001 Jerusalem (IL)
(72) Inventor: DOR, Yuval, 9774415 Jerusalem (IL); SHEMER, Ruth, 9078910 Mevasseret Zion (IL); GLASER, Benjamin, 9692852 Jerusalem (IL); MAGENHEIM, Judith, 9045410 Efrat (IL)
(74) Representative: Murgitroyd & Company

(57) **Abstract**

A method of determining the methylation status of at least one methylation site of a double-stranded DNA molecule is disclosed. The method comprises analyzing the methylation status of both the forward strand and the reverse strand of the DNA molecule.

## Description

### FIELD AND BACKGROUND OF THE INVENTION

The present invention, in some embodiments thereof, relates to detecting tissue specific DNA by analyzing both strands of DNA which is tissue-differentially methylated.

It has been known for decades that plasma contains small fragments of cell-free circulating DNA (cfDNA) derived from dead cells (on average 5000 genome equivalents per ml). While the mechanisms underlying the release and clearance of cfDNA remain obscure, the phenomenon is rapidly being exploited for a variety of applications with clinical relevance. The recognition that fragments of fetal DNA travel briefly in maternal circulation has opened the way for next generation sequencing (NGS)-based prenatal testing to identify fetal trisomies and other genetic aberrations, potentially replacing amniocentesis. In cancer biology, tumors are known to release DNA (including tumor-specific somatic mutations) into the circulation, providing means for liquid biopsies to monitor tumor dynamics and genomic evolution. In addition, cfDNA has been used to detect graft cell death after kidney, liver or heart transplantation, based on single nucleotide polymorphisms (SNPs) distinguishing the DNA of donor from that of recipients. In all these cases, genetic differences exist between the DNA sequence of the tissue of interest (fetus, tumor or graft) and that of the host, providing the basis for highly specific assays.

Blood levels of cfDNA are known to increase under multiple additional conditions such as traumatic brain injury, cardiovascular disease, sepsis and intensive exercise. However in these cases, the source of elevated cfDNA is unknown, greatly compromising the utility of cfDNA as a diagnostic or prognostic tool. For example, cfDNA could originate from parenchymal cells of the injured tissue, but also from dying inflammatory cells.

Despite having an identical nucleotide sequence, the DNA of each cell type in the body carries unique epigenetic marks correlating with its gene expression profile. In particular, DNA methylation, serving to repress nontranscribed genes, is a fundamental aspect of tissue identity. Methylation patterns are unique to each cell type, conserved among cells of the same type in the same individual and between individuals, and are highly stable under physiologic or pathologic conditions. Therefore, it may be possible to use the DNA methylation pattern of cfDNA to determine its tissue of origin and hence to infer cell death in the source organ.

Theoretically, such an approach could identify the rate of cell death in a tissue of interest, taking into account the total amount of cfDNA, the fraction derived from a tissue of interest, and the estimated half-life of cfDNA (15-120 minutes). Note that since the approach relies on normal, stable markers of cell identity, it cannot identify the nature of the pathology (e.g. distinguishing cfDNA derived from dead tumor cells or dead wild type cells due to trauma or inflammation in the same tissue). The potential uses of a highly sensitive, minimally invasive assay of tissue specific cell death include early, precise diagnosis as well as monitoring response to therapy in both a clinical and drug-development setting.

A classic example of tissue-specific DNA methylation is provided by the insulin gene promoter, which is unmethylated in insulin-producing pancreatic β-cells and methylated elsewhere. Recent studies have identified unmethylated insulin promoter DNA in the circulation of newly diagnosed T1D patients as well as in islet graft recipients, likely reflecting both autoimmune and alloimmune destruction of β -cells (Akirav E.M.et al. Proceedings of the National Academy of Sciences of the United States of America, 108, 19018-19023 (2011); Lebastchi J et al., Diabetes 62, 1676-1680 (2013); Husseiny M. I. Plos one 9 e94591 (2014; and Herold K.C. et al., J Clin Invest. Doi:10.1172/jc178142 (2015)).

Additional background art includes Bidshahri et al., The Journal of Molecular Diagnostics, Vol. 18, No. 2, March 2016, Usmani-Brown et al., Endocrinology 155: 3694-3698, 2014; International PCT Publication No. WO2013131083, WO 2014138133, WO201101728, WO2015/159292 and WO2015169947.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

### SUMMARY OF THE INVENTION

According to an aspect of some embodiments of the present invention there is provided a method of determining the methylation status of at least one methylation site of a double-stranded DNA molecule, the method comprising:
(a) contacting the double-stranded DNA with bisulfite to generate:
   (i) a forward single-stranded DNA molecule of which demethylated cytosines of the single-stranded DNA molecules are converted to uracils and;
   (ii) a reverse single-stranded DNA molecule of which demethylated cytosines of the single-stranded DNA molecules are converted to uracils; and
(b) determining the methylation status of the at least one methylation site on the forward strand;
(c) determining the methylation status of the at least one methylation site on the reverse strand, wherein a methylation status of the at least one methylation site on the forward strand and the at least one methylation site on the reverse strand is indicative of the methylation status of the methylation site.

According to an aspect of some embodiments of the present invention there is provided a kit for determining the methylation status of at least one methylation site of a double-stranded DNA molecule, the kit comprising:
(i) a set of primers that are capable of amplifying the forward strand of the double-stranded DNA molecule and not the reverse strand of the double-stranded DNA molecule;
(ii) a set of primers that are capable of amplifying the reverse strand of the double-stranded DNA molecule and not the forward strand of the double-stranded DNA molecule.

According to some embodiments of the invention, the double-stranded DNA molecule is no longer than 300 base pairs.

According to some embodiments of the invention, the double-stranded DNA molecule is no longer than 150 base pairs.

According to some embodiments of the invention, the double stranded DNA comprises at least two methylation sites per single strand of the double-stranded DNA molecule.

According to some embodiments of the invention, the at least two methylation sites are not more than 300 bp apart.

According to some embodiments of the invention, the at least two methylation sites are not more than 150 bp apart.

According to some embodiments of the invention, each strand of the double-stranded DNA comprises at least three methylation sites.

According to some embodiments of the invention, the at least three methylation sites are not more than 300 bp apart.

According to some embodiments of the invention, the at least three methylation sites are not more than 150 bp apart.

According to some embodiments of the invention, the method further comprises amplifying the single-stranded DNA molecule following step (a) and prior to step (b).

According to some embodiments of the invention, the method is for determining the cell or tissue of origin of the double-stranded DNA molecule.

According to some embodiments of the invention, the double-stranded DNA molecule is differentially methylated in a cell or tissue of interest.

According to some embodiments of the invention, the cell of interest is selected from the group consisting of a pancreatic beta cell, a pancreatic exocrine cell, a hepatocyte, a brain cell, a lung cell, a uterus cell, a kidney cell, a breast cell, an adipocyte, a colon cell, a rectum cell, a cardiomyocyte, a skeletal muscle cell, a prostate cell and a thyroid cell.

According to some embodiments of the invention, the tissue is selected from the group consisting of pancreatic tissue, liver tissue, lung tissue, brain tissue, uterus tissue, renal tissue, breast tissue, fat, colon tissue, rectum tissue, cardiac tissue, skeletal muscle tissue, prostate tissue and thyroid tissue.

According to some embodiments of the invention, the tissue is cardiac tissue.

According to some embodiments of the invention, the double-stranded DNA molecule is non-methylated in cells of cardiac tissue and methylated in leukocytes.

According to some embodiments of the invention, the double-stranded DNA molecule comprises at least a part of the sequence of human chromosome 12, between coordinates124692462-124692551.

According to some embodiments of the invention, the double-stranded DNA molecule comprises a sequence which is comprised in SEQ ID NOs: 56 or 57.

According to some embodiments of the invention, the determining of steps (b) and (c) is effected using strand-specific oligonucleotides.

According to some embodiments of the invention, the method further comprises sequencing the forward strand and the reverse strand.

According to some embodiments of the invention, the steps (b) and (c) are carried out concomitantly in a single reaction vessel.

According to some embodiments of the invention, the steps (b) and (c) are carried out in separate reaction vessels.

According to some embodiments of the invention, the step (b) and/or step (c) is effected using digital droplet PCR.

According to some embodiments of the invention, the double-stranded DNA is cell-free DNA.

According to some embodiments of the invention, the double-stranded DNA is cellular DNA.

According to some embodiments of the invention, the method further comprises lysing the cells of the cellular DNA prior to the determining.

According to some embodiments of the invention, the molecule is comprised in a body fluid sample.

According to some embodiments of the invention, the body fluid is selected from the group consisting of blood, plasma, sperm, milk, urine, saliva and cerebral spinal fluid.

According to some embodiments of the invention, the body fluid sample comprises DNA from a plurality of cell-types.

According to some embodiments of the invention, the sample is a blood sample.

According to some embodiments of the invention, the method further comprises quantitating the amount of DNA of the cell or tissue origin.

According to some embodiments of the invention, the kit further comprises bisulfite.

According to some embodiments of the invention, the double-stranded DNA molecule is differentially methylated in a first cell of interest with respect to a second cell which is non-identical to the first cell of interest.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

### BRIEF DESCRIPTION OF THE SEVERAL VIEWS OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:

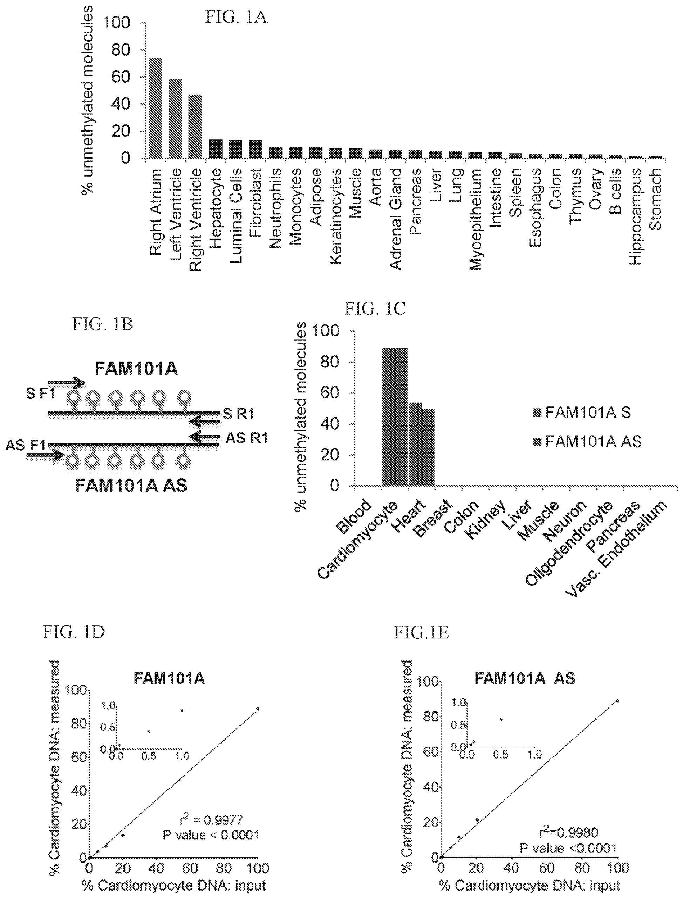

FIGs. 1A-E: Identification of cardiomyocyte-specific DNA methylation markers.
   1A. Unmethylation levels of *FAM101A* locus in 27 human tissues, including left ventricle, right ventricle and right atrium (red). Data was extracted from the Roadmap Epigenomics Consortium browser.
   1B. Structure of the *FAM101A* locus, used as two independent markers: FAM101A and FAM101A AS. Lollipops represent CpG sites; arrows mark positions of PCR primers; S, sense marker; AS, antisense marker.
   1C. Unmethylation status of FAM101A and FAM101A AS in DNA from multiple tissues and from isolated cardiomyocytes (purchased from ScienCell Research Laboratories, San Diego, CA). Targeted PCR yields a lower background in non- cardiac tissues compared with the Roadmap browser in panel A, since the roadmap data includes molecules that contain only some of the cytosines in the FAM101A locus (e.g. only one or two), which can occasionally be demethylated in non-cardiac tissue. In contrast, the targeted PCR by definition amplifies only molecules containing all cytosines in the locus.
   1D-E. Spike in experiments for FAM101A and FAM101A AS. Human cardiomyocyte DNA was mixed with human leukocyte DNA in the indicated proportions (0- 100%), and the percentage of fully unmethylated *FAM101A* molecules (in which all five CpG sites were converted by bisulfite) was determined.

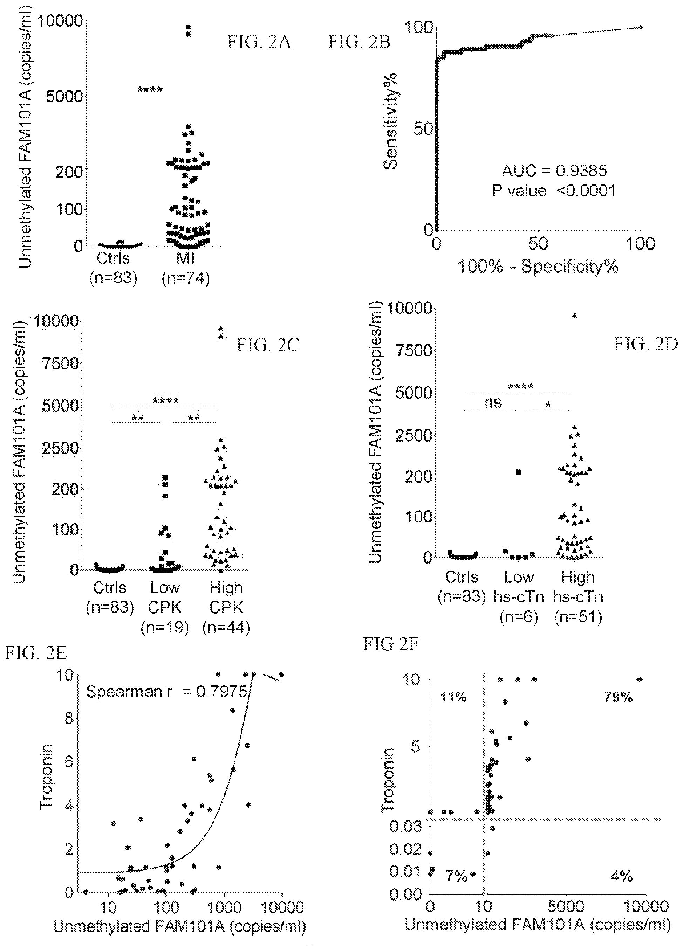

FIGs. 2A-F: Cardiomyocyte-derived cfDNA in healthy subjects and in patients with myocardial infarction.
   A. Cardiac cfDNA (copies of fully unmethylated FAM101A /ml plasma) in samples from healthy controls (n=61) and patients during MI (n=79). Mann-Whitney test for controls vs. patients, *P* <0.0001
   B. Receiver operating characteristic (ROC) curve for unmethylated FAM101A levels in healthy controls and patients with MI. Area under the curve (AUC) 0.884 (95% CI=0.8925 to 0.9766)
   C. Comparison of unmethylated FAM101A levels (copies/ml) in samples from healthy controls, MI patients with low Creatine Kinase (CPK <200) and MI patients with high CK (CK>200). Kruskal-Wallis test P value<0.0001. Dunn's multiple comparisons test adjusted P Value: Ctrls vs. low CK, p< 0.001; Ctrls vs. high CK, P<0.0001; low CK vs. high CK, P=0.0064.
   D. Comparison of unmethylated FAM101A levels in healthy controls, samples with low levels of high-sensitive troponin T (hs-cTn) (<0.03), and samples with high levels of hs-cTn (>0.03).Dunn's multiple comparisons testAdjusted P Value: Ctrls vs. low hs-cTn (<0.03), P=0.8645; Ctrls vs. high hs-cTn (>0.03), PV<0.0001; low hs-cTn (<0.03) vs. high hs-cTn (>0.03), P=0.0189.
   E. Spearman correlation between cardiac cfDNA and troponin levels in n=57 samples.
   F. XY Scatter plot for cardiac cfDNA levels vs. cardiac troponin. Quadrants indicate negative and positive hs-Tn, and negative and positive cardiac cfDNA. Numbers indicate the percentage of samples in each quadrant.
FIGs. 3A-C: Cardiac cfDNA dynamics during MI and after angioplasty.
   A. Cardiac cfDNA levels in MI patients before and after PCI.
   B. ROC curve for cardiac cfDNA in healthy individuals versus MI patients prior to intervention.
   C. Time course of cardiac cfDNA and troponin levels in five patients. Vertical dashed lines indicate PCI time.
FIGs. 4A-C: Cardiac cfDNA in sepsis.
   A. Levels of cardiac cfDNA in healthy controls and patients with sepsis.
   B. Lack of correlation between cardiac cfDNA and troponin. Curved line represents non linear (quadratic) fit.
   C.Kaplan-Meier plot showing correlation of cardiac cfDNA to patient survival.
FIGs. 5A-D: detection of cardiac cfDNA using digital droplet PCR.
   A. Schematic of approach for ddPCR-based detection of methylation status of multiple adjacent cytosines. A signal from two probes in the same droplet reflects lack of methylation in 5 adjacent cytosines in the same original DNA strand.
   B. Signal from cardiomyocyte and leukocyte DNA based on individual or dual probes. Scoring only dual probe signals drastically reduces noise from leukocyte DNA.
   C. Spike-in experiment assessing sensitivity and linearity of signal from cardiomyocyte DNA diluted in leukocyte DNA. The use of dual probe enhances linearity and reduces baseline signal.
   D. Measurement of cardiac cfDNA in plasma of healthy adult and patients with myocardial infarction. The use of dual probes reduces the baseline signal in healthy plasma.

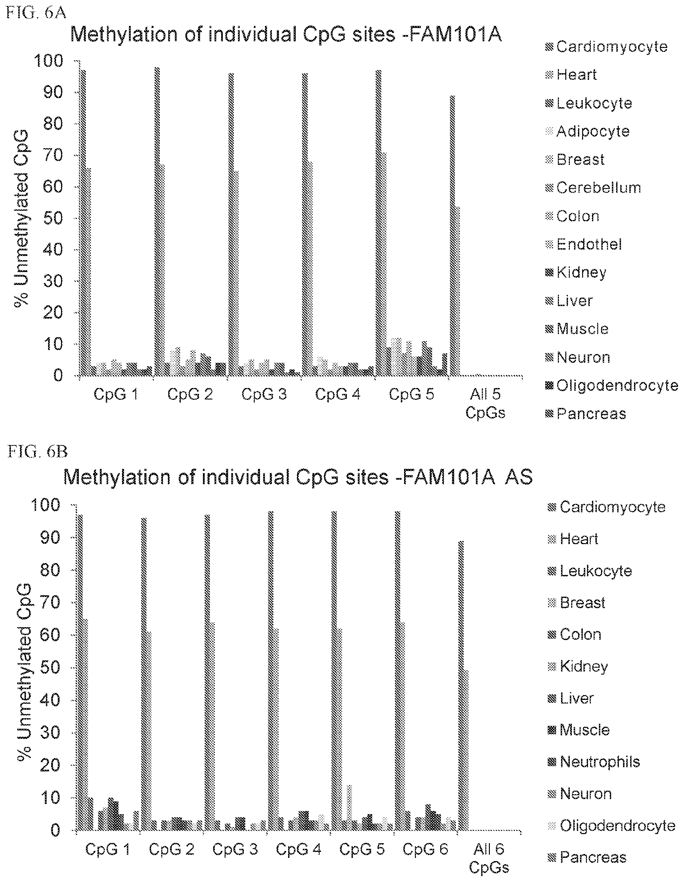

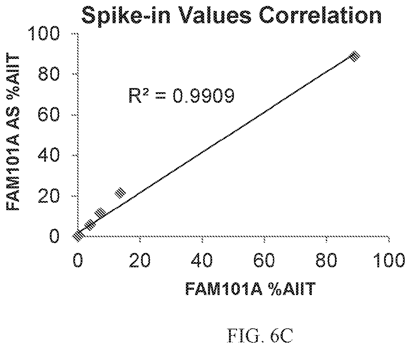

FIGs. 6A-C: methylation of individual and multiple adjacent cytosines within the FAM101A locus.
   A. Methylation status of cytosines in the sense strand of FAM101A
   B. Metylation status of cytosines in the antisense (AS) strand of FAM101A. Graphs shows the percentage of unmethylated molecules in DNA from each tissue. The set of columns on the far right describes the percentage of molecules in which all CpG sites are unmethylated, demonstrating the higher signal-to-noise ratio afforded by interrogating all CpGs simultaneously.
   C. Correlation between results of spike-in experiments using the sense and antisense FAM101A markers.

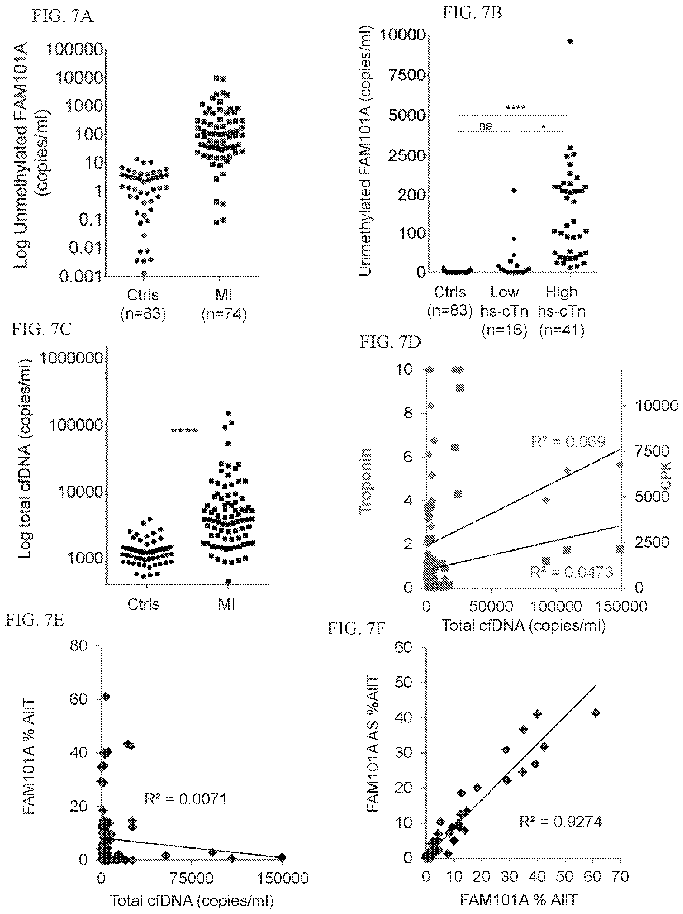

FIGs. 7A-F: additional correlations of cardiac and total cfDNA in MI patients.
   A. Log scale presentation of unmethylated FAM101A levels in plasma samples from healthy controls (n=83) and patients during MI (n=74). 54 values were zero, so are not shown in the graph.
   B. Cardiac cfDNA levels in controls vs MI patients positive or negative for high sensitive troponin using 0.1 as a cutoff. Dunn's multiple comparisons test adjusted P value: Ctrls vs. Low hs-cTn (<0.1), P=0.0433; Ctrls vs. High hs-cTn (>0.1), P<0.0001; Low hs-cTn (<0.1) vs. High hs-cTn (>0.1), P=0.0003.
   C. Total cfDNA concentration in controls and MI patients.
   D. Lack of correlation between total concentration of cfDNA (genome equivalents/ml) and either hs-Tn (blue) or CK (red) levels.
   E. Lack of correlation between total cfDNA (genome equivalents/ml) and percentage of cardiac cfDNA.
   F. Linear correlation between FAM101A sense (S) and antisense (AS) signal in the MI samples.

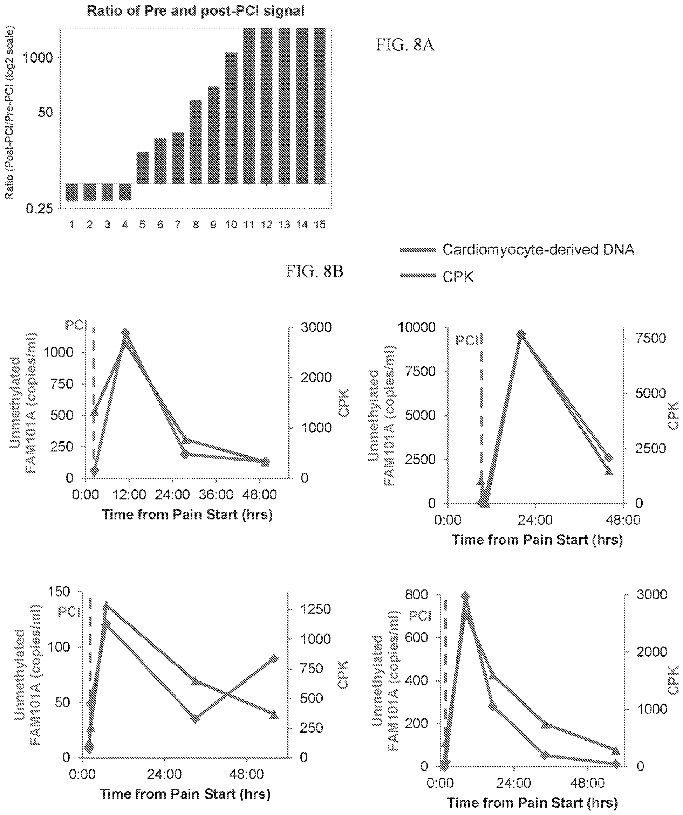

FIGs. 8A-B. Dynamics of cardiac cfDNA and CPK in myocardial infarction.
   A. Ratio of cardiac cfDNA before and after PCI in 15 individuals with MI. As expected, cardiac cfDNA levels increased after intervention.
   B. Dynamics of cardiac cfDNA and CPK in individual patients. Time 0 is the beginning of chest pain. Vertical dashed line indicates time of PCI.

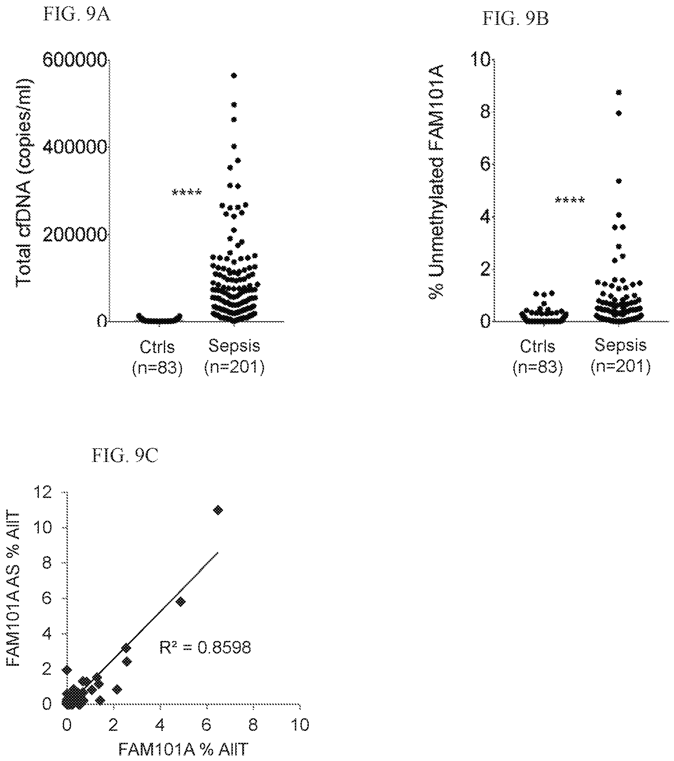

FIGs. 9A-C: Total and cardiac cfDNA levels in patients with sepsis.
   A.Concentration of cfDNA in patients with sepsis.
   B.Percentage of cardiac cfDNA in patients with sepsis.
   C.Correlation between FAM101A sense and antisense signals in sepsis samples.

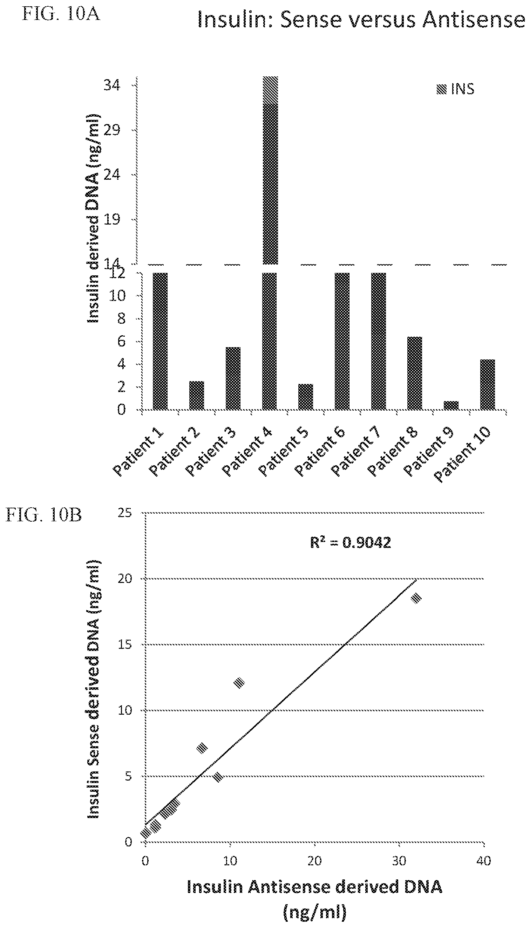

FIGs. 10A-B are graphs illustrating the effectiveness of detecting pancreatic cell cfDNA by detecting both the sense and antisense strand of insulin gene according to embodiments of the present invention.

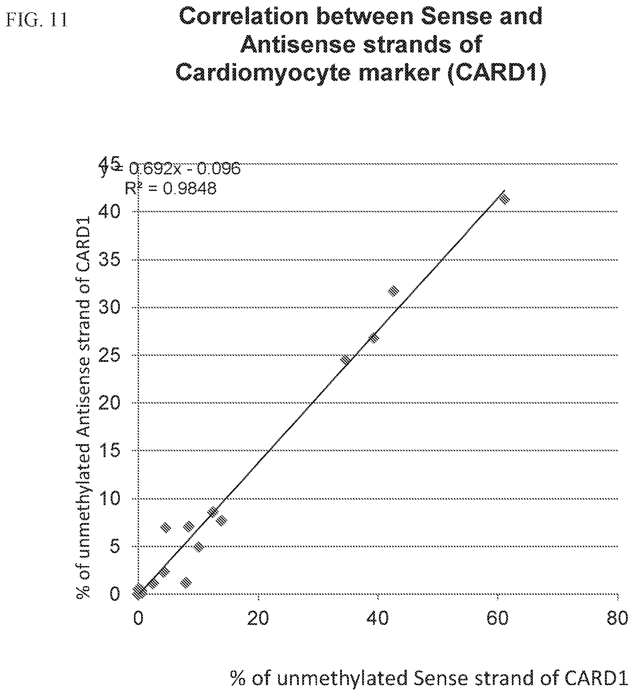

FIG. 11 is a graph illustrating the correlation between Sense and Antisense strands of Cardiomyocyte marker (CARD1).

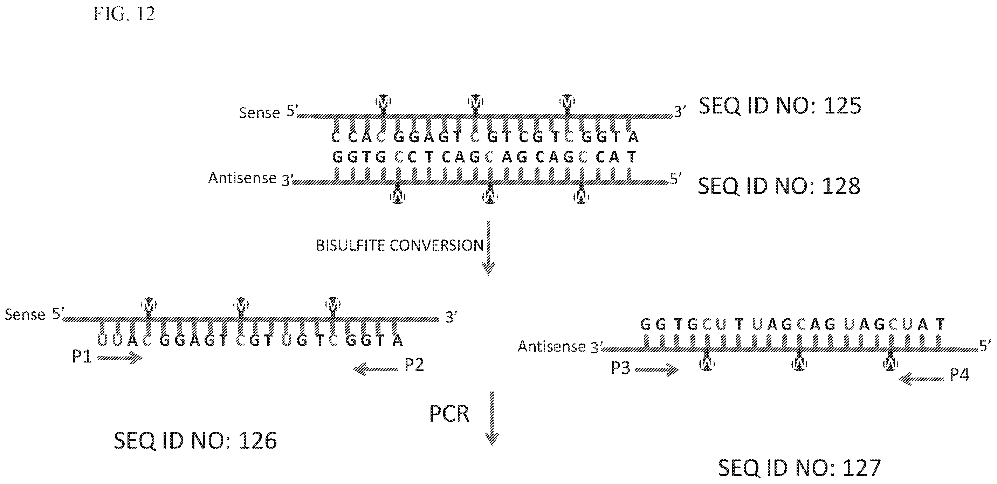

FIG. 12 is a graphic illustrating of a method of analyzing methylation status according to embodiments of the present invention. The bisulfite-converted DNA is single stranded, due to loss of complementarity caused by the replacement of Cs with Us. Therefore primers are designed to be strand specific as well as bisulfite-specific. Since DNA methylation is symmetric, the methylation pattern observed on the sense strand will be complementary to the pattern observed on the antisense strand. Thus primers can be designed to one of the strands or to both of them.

### DESCRIPTION OF SPECIFIC EMBODIMENTS OF THE INVENTION

The present invention, in some embodiments thereof, relates to detecting tissue specific DNA by analyzing both strands of DNA which is tissue-differentially methylated.

Analysis of circulating DNA is beginning to revolutionize prenatal diagnosis, tumor diagnosis and the monitoring of graft rejection. However a major limitation of all applications is the dependence on the presence of identifiable genetic differences between the tissue of interest and the host**.** It has recently been shown that determination of the tissue origins of circulating free DNA (cfDNA) can be carried out by analyzing tissue-specific methylation markers. For every stretch of CpG sites showing a tissue-specific methylation pattern there is a parallel stretch in the opposite strand of DNA. The standard procedure for analysis of methylation involves treatment of DNA with bisultite to convert unmethylated (but not methylated) cytosines to uracils. As a result of bisulfite conversion, the sequences of complementary DNA strands become less similar, such that base pairing does not occur anymore and the DNA becomes single stranded (as illustrated in Figure 12).

Consequently, PCR amplification and analysis (via sequencing or other means) of bisulfite-treated cfDNA typically focus on just one strand of the original double stranded DNA. The other single strand, which contains exactly the same epigenetic information, remains in the solution and is not used. The present inventors realized that after bisulfite treatment of double stranded DNA, each single stranded molecule becomes an independent entity.

Detection of even one of the strands carrying the relevant methylation signature is sufficient to identify DNA even if present in a minute quantities, or mixed with other "noise" DNA- a specific non-limiting example being the detection of cfDNA from a specific tissue or from a specific tumor.

The present inventors have now realized that by analyzing in parallel the two strands of a given tissue-specific methylation signature, the sensitivity for detection of methylated/non-methylated DNA will double. This is particularly important in cases when there are small amounts of the DNA or few probes such as in cases where the tissue-specific cfDNA is rare and difficult to identify. The parallel assessment of sense and antisense markers increase the sensitivity of the methylation assay, and also increases the confidence in the detection of a true signal.

The present inventors developed a procedure for parallel amplification of the two DNA strands from the same fragment, after bisulfite treatment. The procedure is based on two primer pairs, each of which is specific to one of the strands after bisulfite treatment. Using this approach, they were able to detect both strands of a given tissue-specific marker, in cfDNA extracted from plasma. They further showed that this increases the chance of detecting DNA molecules, when dealing with limiting amounts of plasma. As a proof of concept, they showed the utility of parallel measurement of sense and antisense markers of pancreatic beta cells (Figures 10A-B) and of cardiomyocytes (Figure 11).

Thus, according to a first aspect of the present invention there is provided a method of determining the methylation status of at least one methylation site of a double-stranded DNA molecule, the method comprising:
(a) contacting the double-stranded DNA with bisulfite to generate:
   (i) a forward single-stranded DNA molecule of which demethylated cytosines of said single-stranded DNA molecules are converted to uracils and;
   (ii) a reverse single-stranded DNA molecule of which demethylated cytosines of said single-stranded DNA molecules are converted to uracils; and
(b) determining the methylation status of said at least one methylation site on said forward strand;
(c) determining the methylation status of said at least one methylation site on said reverse strand, wherein a methylation status of said at least one methylation site on said forward strand and said at least one methylation site on said reverse strand is indicative of the methylation status of said methylation site.

As used herein, the term "methylation status" refers to the status of a cytosine in a DNA sequence. The cytosine may be methylated (and present as 5-methylcytosine) or non-methylated and present as cytosine.

As used herein, the term "methylation site" refers to a cytosine residue adjacent to guanine residue (CpG site) that has a potential of being methylated.

The DNA molecule is preferably no longer than 300 nucleotides, 295 nucleotides, 290 nucleotides, 285 nucleotides, 280 nucleotides, 275 nucleotides, 270 nucleotides, 265 nucleotides, 260 nucleotides, 255 nucleotides, 250 nucleotides, 245 nucleotides, 240 nucleotides, 235 nucleotides, 230 nucleotides, 225 nucleotides, 220 nucleotides, 215 nucleotides, 210 nucleotides, 205 nucleotides, 200 nucleotides, 195 nucleotides, 190 nucleotides, 185 nucleotides, 180 nucleotides, 175 nucleotides, 170 nucleotides, 165 nucleotides, 160 nucleotides, 155 nucleotides, 150 nucleotides, 145 nucleotides, 140 nucleotides, 135 nucleotides, 130 nucleotides, 125 nucleotides, 120 nucleotides, 115 nucleotides, 110 nucleotides, 105 nucleotides, 100 nucleotides, 95 nucleotides, 90 nucleotides, 85 nucleotides, 80 nucleotides, 75 nucleotides, 70 nucleotides, 65 nucleotides, 60 nucleotides, 55 nucleotides, or 50 nucleotides.

According to a particular embodiment, the DNA molecule is between 50-300 nucleotides, e.g. between 50-250, between 50-200, between 100-300 nucleotides, or between 100-250 nucleotides.

The sequence may be of a coding or non-coding region.

The DNA may be a signal of aberrant methylation such as in the case of a tumor or a disease process.

According to a particular embodiment, the sequence is not derived from a gene which is differentially expressed in the cell of interest. Thus, for example in the case of identifying a methylation pattern for a pancreatic beta cell, the DNA sequence may not be part of a gene encoding insulin or another pancreatic beta cell protein.

In accordance with another particular embodiment, the methylation pattern characterizes the normal cell of interest and is not a methylation pattern characterizing a diseased cell (is not for example a methylation pattern characterizing cancer cells of a specific type).

The method of the present invention contemplates analyzing at least 2, at least 3, at least 4, at least 5, at least 6, at least 7 at least 8, at least 9 or even at least 10 or more methylation sites per double-stranded DNA molecule.

Thus, the methylation signature of the DNA molecule may comprise at least 2, at least 3 comprise at least 4, at least 5, at least 6, at least 7 at least 8, at least 9 or even at least 10 or more methylation sites.

In a particular embodiment, the signature of the DNA molecule does not comprise more than 4 methylation sites per single strand of the DNA molecule.

In a particular embodiment, the signature of the DNA molecule does not comprise more than 3 methylation sites per single strand of the DNA molecule.

In a particular embodiment, the signature of the DNA molecule does not comprise more than 2 methylation sites per single strand of the DNA molecule.

In a particular embodiment, the signature of the DNA molecule does not comprise more than 1 methylation sites per single strand of the DNA molecule.

In the case where there is more than one methylation site per single strand of the DNA molecule, the methylation sites of the signature are no more than 300 nucleotides apart, 295 nucleotides apart, 290 nucleotides apart, 285 nucleotides apart, 280 nucleotides apart, 275 nucleotides apart, 270 nucleotides apart, 265 nucleotides apart, 260 nucleotides apart, 255 nucleotides apart, 250 nucleotides apart, 245 nucleotides apart, 240 nucleotides apart, 235 nucleotides apart, 230 nucleotides apart, 225 nucleotides apart, 220 nucleotides apart, 215 nucleotides apart, 210 nucleotides apart, 205 nucleotides apart, 200 nucleotides apart, 195 nucleotides apart, 190 nucleotides apart, 185 nucleotides apart, 180 nucleotides apart, 175 nucleotides apart, 170 nucleotides apart, 165 nucleotides apart, 160 nucleotides apart, 155 nucleotides apart, 150 nucleotides apart, 145 nucleotides apart, 140 nucleotides apart, 135 nucleotides apart, 130 nucleotides apart, 125 nucleotides apart, 120 nucleotides apart, 115 nucleotides apart, 110 nucleotides apart, 105 nucleotides apart, 100 nucleotides apart, 95 nucleotides apart, 90 nucleotides apart, 85 nucleotides apart, 80 nucleotides apart, 75 nucleotides apart, 70 nucleotides apart, 65 nucleotides apart, 60 nucleotides apart, 55 nucleotides apart, or 50 nucleotides apart.

In order to be considered a methylation signature for a particular cell of interest each of the methylation sites of the signature on the DNA molecule should be differentially methylated in that cell of interest with respect to a second non-identical cell. The methylation signature reflects the methylation status of at least two, at least three, at least four methylation sites of a particular DNA molecule. The methylation sites of the signature may be on a single strand of the DNA molecule or distributed amongst both strands of the DNA molecule.

According to a particular embodiment, each of the at least one, two, three or four methylation sites of the signature are unmethylated in the cell of interest (the cell for which the methylation pattern is being determined) on the DNA molecule, whereas in the second non-identical cell each of the sites are methylated on the DNA molecule.

According to another embodiment, each of the at least one, two, three or four methylation sites of the signature are methylated in the cell of interest on the DNA molecule, whereas in the second non-identical cell each of the sites are unmethylated on the DNA molecule.

According to another embodiment, at least one of the methylation sites of the signature is unmethylated in the cell of interest on the DNA molecule, whereas in the second non-identical cell that site is methylated on the DNA molecule.

According to another embodiment, at least one of the methylation sites of the signature is methylated in the cell of interest on the DNA molecule, whereas in the second non-identical cell that site is unmethylated on the DNA molecule.

According to another embodiment, at least two methylation sites of the signature are unmethylated in the cell of interest on the DNA molecule, whereas in the second non-identical cell those sites are methylated on the DNA molecule.

According to another embodiment, at least two methylation sites of the signature are methylated in the cell of interest on the DNA molecule, whereas in the second non-identical cell those sites are unmethylated on the DNA molecule.

According to another embodiment, at least three methylation sites of the signature are unmethylated in the cell of interest on the DNA molecule, whereas in the second non-identical cell those sites are methylated on the DNA molecule.

According to another embodiment, at least three methylation sites of the signature are methylated in the cell of interest on the DNA molecule, whereas in the second non-identical cell those sites are unmethylated on the DNA molecule.

According to another embodiment, at least four methylation sites of the signature are unmethylated in the cell of interest on the DNA molecule, whereas in the second non-identical cell those sites are methylated on the DNA molecule.

According to another embodiment, at least four methylation sites of the signature are methylated in the cell of interest on the DNA molecule, whereas in the second non-identical cell those sites are unmethylated on the DNA molecule.

The second non-identical cell may be of any source including for example blood cells. Typically, the non-identical cell is one which is comprised in the specimen/sample being analyzed.

The method can be used for identifying methylation signatures of any cell of interest, including but not limited to cardiac cells (e.g. cardiomyocytes), pancreatic cells (such as pancreatic beta cells, exocrine pancreatic cells (e.g. acinar cells), brain cells, oligodendrocytes, liver cells (hepatocytes), kidney cells, tongue cells, vascular endothelial cells, lymphocytes, neutrophils, melanocytes, T-regs, lung cells, a uterus cells, breast cells, adipocytes, colon cells, rectum cells, prostate cells, thyroid cells and skeletal muscle cells.

Specimens which may be analyzed are generally fluid samples, for example body fluids derived from mammalian subjects and include for example blood, plasma, sperm, milk, urine, saliva or cerebral spinal fluid. Alternatively, the specimens may be derived from biopsies.

According to a particular embodiment, the specimen is plasma or blood.

Specimens which are analyzed typically comprise DNA from at least one, or at least two cell/tissue sources, as further described herein below. Thus for example the specimens may comprise cell-free DNA from a single cell type, two cell types or more than two cell types.

According to one embodiment, a sample of blood is obtained from a subject according to methods well known in the art. Plasma or serum may be isolated according to methods known in the art.

DNA may be isolated from the blood immediately or within 1 hour, 2 hours, 3 hours, 4 hours, 5 hours or 6 hours. Optionally the blood is stored at temperatures such as 4 °C, or at -20 °C prior to isolation of the DNA. In some embodiments, a portion of the blood sample is used in accordance with the invention at a first instance of time whereas one or more remaining portions of the blood sample (or fractions thereof) are stored for a period of time for later use.

According to one embodiment, the DNA which is analyzed is cellular DNA (i.e. comprised in a cell).

According to still another embodiment, the DNA which is analyzed is comprised in a shedded cell or non-intact cell.

Methods of DNA extraction are well-known in the art. A classical DNA isolation protocol is based on extraction using organic solvents such as a mixture of phenol and chloroform, followed by precipitation with ethanol (J. Sambrook et al., "Molecular Cloning: A Laboratory Manual", 1989, 2nd Ed., Cold Spring Harbour Laboratory Press: New York, N.Y.). Other methods include: salting out DNA extraction (P. Sunnucks et al., Genetics, 1996, 144: 747-756; S. M. Aljanabi and I. Martinez, Nucl. Acids Res. 1997, 25: 4692-4693), trimethylammonium bromide salts DNA extraction (S. Gustincich et al., BioTechniques, 1991, 11: 298-302) and guanidinium thiocyanate DNA extraction (J. B. W. Hammond et al., Biochemistry, 1996, 240: 298-300).

There are also numerous versatile kits that can be used to extract DNA from tissues and bodily fluids and that are commercially available from, for example, BD Biosciences Clontech (Palo Alto, Calif.), Epicentre Technologies (Madison, Wis.), Gentra Systems, Inc. (Minneapolis, Minn.), MicroProbe Corp. (Bothell, Wash.), Organon Teknika (Durham, N.C.), and Qiagen Inc. (Valencia, Calif.). User Guides that describe in great detail the protocol to be followed are usually included in all these kits. Sensitivity, processing time and cost may be different from one kit to another. One of ordinary skill in the art can easily select the kit(s) most appropriate for a particular situation.

According to another embodiment, the DNA which is analyzed is cell-free DNA. For this method, cell lysis is not performed on the sample. Methods of isolating cell-free DNA from body fluids are also known in the art. For example Qiaquick kit, manufactured by Qiagen may be used to extract cell-free DNA from plasma or serum.

The sample may be processed before the method is carried out, for example DNA purification may be carried out following the extraction procedure. The DNA in the sample may be cleaved either physically or chemically (e.g. using a suitable enzyme). Processing of the sample may involve one or more of: filtration, distillation, centrifugation, extraction, concentration, dilution, purification, inactivation of interfering components, addition of reagents, and the like.

To analyze methylation status according to this aspect of the present invention, the DNA is treated with bisulfite which converts cytosine residues to uracil (which are converted to thymidine following PCR), but leaves 5-methylcytosine residues unaffected. Thus, bisulfite treatment introduces specific changes in the DNA sequence that depend on the methylation status of individual cytosine residues, yielding single- nucleotide resolution information about the methylation status of a segment of DNA.

During the bisulfite reaction, care should be taken to minimize DNA degradation, such as cycling the incubation temperature.

Bisulfite sequencing relies on the conversion of every single unmethylated cytosine residue to uracil. If conversion is incomplete, the subsequent analysis will incorrectly interpret the unconverted unmethylated cytosines as methylated cytosines, resulting in false positive results for methylation. Only cytosines in single-stranded DNA are susceptible to attack by bisulfite, therefore denaturation of the DNA undergoing analysis is critical. It is important to ensure that reaction parameters such as temperature and salt concentration are suitable to maintain the DNA in a single-stranded conformation and allow for complete conversion.

According to a particular embodiment, an oxidative bisulfite reaction is performed. 5-methylcytosine and 5-hydroxymethylcytosine both read as a C in bisulfite sequencing. Oxidative bisulfite reaction allows for the discrimination between 5-methylcytosine and 5-hydroxymethylcytosine at single base resolution. The method employs a specific chemical oxidation of 5-hydroxymethylcytosine to 5-formylcytosine, which subsequently converts to uracil during bisulfite treatment. The only base that then reads as a C is 5-methylcytosine, giving a map of the true methylation status in the DNA sample. Levels of 5-hydroxymethylcytosine can also be quantified by measuring the difference between bisulfite and oxidative bisulfite sequencing.

As a result of bisulfite conversion, the sequences of complementary DNA strands become less similar, such that base pairing does not occur anymore and the DNA becomes single stranded.

Thus, following bisulfite treatment, two strands of non-complementary DNA are generated:
(i) a forward single-stranded DNA molecule of which demethylated cytosines of the single-stranded DNA molecules are converted to uracils and;
(ii) a reverse single-stranded DNA molecule of which demethylated cytosines of the single-stranded DNA molecules are converted to uracils.

The methylation pattern of each of the single-stranded DNA molecules is then analyzed individually.

Optionally, the bisulfite-treated DNA molecules are subjected to an amplification reaction prior to, or concomitant with, analysis of the methylation pattern.

As used herein, the term "amplification" refers to a process that increases the representation of a population of specific nucleic acid sequences in a sample by producing multiple (i.e., at least 2) copies of the desired sequences. Methods for nucleic acid amplification are known in the art and include, but are not limited to, polymerase chain reaction (PCR) and ligase chain reaction (LCR). In a typical PCR amplification reaction, a nucleic acid sequence of interest is often amplified at least fifty thousand fold in amount over its amount in the starting sample. A "copy" or "amplicon" does not necessarily mean perfect sequence complementarity or identity to the template sequence. For example, copies can include nucleotide analogs such as deoxyinosine, intentional sequence alterations (such as sequence alterations introduced through a primer comprising a sequence that is hybridizable but not complementary to the template), and/or sequence errors that occur during amplification.

A typical amplification reaction is carried out by contacting a forward and reverse primer (a primer pair) to the sample DNA together with any additional amplification reaction reagents under conditions which allow amplification of the target sequence. The oligonucleotide amplification primers typically flank the target sequence - (i.e. the sequence comprising the at least one, two, three, four or five methylation sites (per single strand).

The terms "forward primer" and "forward amplification primer" are used herein interchangeably, and refer to a primer that hybridizes (or anneals) to the target (template strand). The terms "reverse primer" and "reverse amplification primer" are used herein interchangeably, and refer to a primer that hybridizes (or anneals) to the complementary target strand. The forward primer hybridizes with the target sequence 5' with respect to the reverse primer.

It will be appreciated that two amplification reactions are performed, one on the forward strand and one of the reverse strand. Thus, the present inventors contemplate use of strand-specific oligonucleotides (either primers or probes as further described herein below).

The two amplification reactions may be carried out concomitantly (e.g. in the same reaction vessel, at the same time - multiplex reaction) or consecutively.

The term "amplification conditions", as used herein, refers to conditions that promote annealing and/or extension of primer sequences. Such conditions are well-known in the art and depend on the amplification method selected. Thus, for example, in a PCR reaction, amplification conditions generally comprise thermal cycling, i.e., cycling of the reaction mixture between two or more temperatures. In isothermal amplification reactions, amplification occurs without thermal cycling although an initial temperature increase may be required to initiate the reaction. Amplification conditions encompass all reaction conditions including, but not limited to, temperature and temperature cycling, buffer, salt, ionic strength, and pH, and the like.

As used herein, the term "amplification reaction reagents", refers to reagents used in nucleic acid amplification reactions and may include, but are not limited to, buffers, reagents, enzymes having reverse transcriptase and/or polymerase activity or exonuclease activity, enzyme cofactors such as magnesium or manganese, salts, nicotinamide adenine dinuclease (NAD) and deoxynucleoside triphosphates (dNTPs), such as deoxyadenosine triphospate, deoxyguanosine triphosphate, deoxycytidine triphosphate and thymidine triphosphate. Amplification reaction reagents may readily be selected by one skilled in the art depending on the amplification method used.

The present inventors contemplate fractionating the DNA from the sample/specimen prior to performing an amplification reaction. In one embodiment, the amplification reaction is a digital droplet PCR reaction (ddPCR).

To fractionate the DNA sample/specimen, eemulsification techniques can be used so as to create large numbers of aqueous droplets that function as independent reaction chambers for the PCR reactions. For example, an aqueous specimen (e.g., 20 microliters) can be partitioned into droplets (e.g., 20,000 droplets of one nanoliter each) to allow an individual test for the target to be performed with each of the droplets.

Aqueous droplets can be suspended in oil to create a water-in-oil emulsion (W/O). The emulsion can be stabilized with a surfactant to reduce coalescence of droplets during heating, cooling, and transport, thereby enabling thermal cycling to be performed.

In an exemplary droplet-based digital assay, a specimen is partitioned into a set of droplets at a dilution that ensures that more than 40% of the droplets contain no more than one single-stranded DNA molecule per specimen fraction.

In an exemplary droplet-based digital assay, a specimen is partitioned into a set of droplets at a dilution that ensures that more than 50% of the droplets contain no more than one single-stranded DNA molecule per specimen fraction.

In an exemplary droplet-based digital assay, a specimen is partitioned into a set of droplets at a dilution that ensures that more than 60% of the droplets contain no more than one single-stranded DNA molecule per specimen fraction.

In an exemplary droplet-based digital assay, a specimen is partitioned into a set of droplets at a dilution that ensures that more than 70% of the droplets contain no more than one single-stranded DNA molecule per specimen fraction.

In an exemplary droplet-based digital assay, a specimen is partitioned into a set of droplets at a dilution that ensures that more than 80% of the droplets contain no more than one single-stranded DNA molecule per specimen fraction.

In an exemplary droplet-based digital assay, a specimen is partitioned into a set of droplets at a dilution that ensures that more than 90% of the droplets contain no more than one single-stranded DNA molecule per specimen fraction.

Once fractionation has taken place, the single-stranded DNA may then optionally be amplified.

Whether subjected to fractionation or not, the primers which are used in the amplification reaction may be methylation independent primers. These primers flank the first and last of the at least four methylation sites (but do not hybridize directly to the sites) and in a PCR reaction, are capable of generating an amplicon which comprises the methylation sites of the methylation signature.

The methylation-independent primers may comprise adaptor sequences which include barcode sequences. The adaptors may further comprise sequences which are necessary for attaching to a flow cell surface (P5 and P7 sites, for subsequent sequencing), a sequence which encodes for a promoter for an RNA polymerase and/or a restriction site. The barcode sequence may be used to identify a particular molecule, sample or library. The barcode sequence may be between 3-400 nucleotides, more preferably between 3-200 and even more preferably between 3-100 nucleotides. Thus, the barcode sequence may be 6 nucleotides, 7 nucleotides, 8, nucleotides, nine nucleotides or ten nucleotides. The barcode is typically 4-15 nucleotides.

When methylation independent primers are used to amplify the target sequences, the sequence of the target sequence may be uncovered using sequencing techniques known in the art - e.g. massively parallel DNA sequencing, sequencing-by-synthesis, sequencing-by-ligation, 454 pyrosequencing, cluster amplification, bridge amplification, and PCR amplification, although preferably, the method comprises a high throughput sequencing method. Typical methods include the sequencing technology and analytical instrumentation offered by Roche 454 Life Sciences^{™}, Branford, Conn., which is sometimes referred to herein as "454 technology" or "454 sequencing."; the sequencing technology and analytical instrumentation offered by Illumina, Inc, San Diego, Calif. (their Solexa Sequencing technology is sometimes referred to herein as the "Solexa method" or "Solexa technology"); or the sequencing technology and analytical instrumentation offered by ABI, Applied Biosystems, Indianapolis, Ind., which is sometimes referred to herein as the ABI-SOLiD^{™} platform or methodology.

Other known methods for sequencing include, for example, those described in: Sanger, F. et al., Proc. Natl. Acad. Sci. U.S.A. 75, 5463-5467 (1977); Maxam, A. M. & Gilbert, W. Proc Natl Acad Sci USA 74, 560-564 (1977); Ronaghi, M. et al., Science 281, 363, 365 (1998); Lysov, l. et al., Dokl Akad Nauk SSSR 303, 1508-1511 (1988); Bains W. & Smith G. C. J. Theor Biol 135, 303-307 (1988); Drnanac, R. et al., Genomics 4, 114-128 (1989); Khrapko, K. R. et al., FEBS Lett 256.118-122 (1989); Pevzner P. A. J Biomol Struct Dyn 7, 63-73 (1989); and Southern, E. M. et al., Genomics 13, 1008-1017 (1992). Pyrophosphate-based sequencing reaction as described, e.g., in U.S. Patent Nos. 6,274,320, 6,258,568 and 6,210,891, may also be used.

The Illumina or Solexa sequencing is based on reversible dye-terminators. DNA molecules are typically attached to primers on a slide and amplified so that local clonal colonies are formed. Subsequently one type of nucleotide at a time may be added, and non-incorporated nucleotides are washed away. Subsequently, images of the fluorescently labeled nucleotides may be taken and the dye is chemically removed from the DNA, allowing a next cycle. The Applied Biosystems' SOLiD technology, employs sequencing by ligation. This method is based on the use of a pool of all possible oligonucleotides of a fixed length, which are labeled according to the sequenced position. Such oligonucleotides are annealed and ligated. Subsequently, the preferential ligation by DNA ligase for matching sequences typically results in a signal informative of the nucleotide at that position. Since the DNA is typically amplified by emulsion PCR, the resulting bead, each containing only copies of the same DNA molecule, can be deposited on a glass slide resulting in sequences of quantities and lengths comparable to Illumina sequencing. Another example of an envisaged sequencing method is pyrosequencing, in particular 454 pyrosequencing, e.g. based on the Roche 454 Genome Sequencer. This method amplifies DNA inside water droplets in an oil solution with each droplet containing a single DNA template attached to a single primer-coated bead that then forms a clonal colony. Pyrosequencing uses luciferase to generate light for detection of the individual nucleotides added to the nascent DNA, and the combined data are used to generate sequence read-outs. A further method is based on Helicos' Heliscope technology, wherein fragments are captured by polyT oligomers tethered to an array. At each sequencing cycle, polymerase and single fluorescently labeled nucleotides are added and the array is imaged. The fluorescent tag is subsequently removed and the cycle is repeated. Further examples of sequencing techniques encompassed within the methods of the present invention are sequencing by hybridization, sequencing by use of nanopores, microscopy-based sequencing techniques, microfluidic Sanger sequencing, or microchip-based sequencing methods. The present invention also envisages further developments of these techniques, e.g. further improvements of the accuracy of the sequence determination, or the time needed for the determination of the genomic sequence of an organism etc.

According to one embodiment, the sequencing method comprises deep sequencing.

As used herein, the term "deep sequencing" and variations thereof refers to the number of times a nucleotide is read during the sequencing process. Deep sequencing indicates that the coverage, or depth, of the process is many times larger than the length of the sequence under study.

It will be appreciated that any of the analytical methods described herein can be embodied in many forms. For example, it can be embodied on a tangible medium such as a computer for performing the method operations. It can be embodied on a computer readable medium, comprising computer readable instructions for carrying out the method operations. It can also be embodied in electronic device having digital computer capabilities arranged to run the computer program on the tangible medium or execute the instruction on a computer readable medium.

Computer programs implementing the analytical method of the present embodiments can commonly be distributed to users on a distribution medium such as, but not limited to, CD-ROMs or flash memory media. From the distribution medium, the computer programs can be copied to a hard disk or a similar intermediate storage medium. In some embodiments of the present invention, computer programs implementing the method of the present embodiments can be distributed to users by allowing the user to download the programs from a remote location, via a communication network, *e.g.*, the internet. The computer programs can be run by loading the computer instructions either from their distribution medium or their intermediate storage medium into the execution memory of the computer, configuring the computer to act in accordance with the method of this invention. All these operations are well-known to those skilled in the art of computer systems.

The present invention also contemplates use of methylation-sensitive oligomers as probes. The probes can be added during the amplification reaction (e.g. in a digital droplet PCR (ddPCR) reaction.

In one embodiment, the amplification reaction includes a single labeled oligonucleotide probe which hybridizes to one strand of the amplified double-stranded DNA which comprises the methylation site. Thus, altogether the amplification reaction may include two labeled olignonucleotide probes - one which hybridizes to one strand of the amplified double-stranded DNA which comprises the methylation site originating from the forward strand of the original DNA and one which hybridizes to one strand of the amplified double-stranded DNA which comprises the methylation site originating from the reverse strand of the original DNA.

If the methylation sites are close enough together on the DNA, it is conceivable that the probes of this aspect of the present invention hybridize to more than one methylation site per ssDNA molecule, for example, two, three, or even four.

The sequence of the first and/or second probe may be selected such that it binds to the amplified DNA when the methylation site of the double-stranded DNA molecule is non-methylated.

Alternatively, the sequence of the first and/or second probe may be selected such that it binds to the amplified DNA when the methylation site of the double-stranded DNA molecule is methylated.

The fluorescence signal is directly proportional to DNA concentration over a broad range, and the linear correlation between PCR product and fluorescence intensity is used to calculate the amount of template DNA (comprising the target nucleic acid sequence) present at the beginning of the reaction. The point at which fluorescence is first detected as statistically significant above the baseline or background, is called the threshold cycle or Ct Value. The Ct Value is the most important parameter for quantitative PCR. This threshold must be established to quantify the amount of DNA in the samples. It is inversely correlated to the logarithm of the initial copy number. The threshold should be set above the amplification baseline and within the exponential increase phase (which looks linear in the log phase). Most assay systems automatically calculate the threshold level of fluorescence signal by determining the baseline (background) average signal and setting a threshold 10-fold higher than this average.

Preferably, when more than one probe is used in any of the amplification reactions described herein above, the probes are labeled with non-identical labels i.e. detectable moieties.

The oligonucleotides of the invention need not reflect the exact sequence of the target nucleic acid sequence (i.e. need not be fully complementary), but must be sufficiently complementary so as to hybridize to the target site under the particular experimental conditions. Accordingly, the sequence of the oligonucleotide typically has at least 70 % homology, preferably at least 80 %, 90 %, 95 %, 97 %, 99 % or 100 % homology, for example over a region of at least 13 or more contiguous nucleotides with the target sequence. The conditions are selected such that hybridization of the oligonucleotide to the target site is favored and hybridization to the non-target site is minimized.

Various considerations must be taken into account when selecting the stringency of the hybridization conditions. For example, the more closely the oligonucleotide (e.g. primer) reflects the target nucleic acid sequence, the higher the stringency of the assay conditions can be, although the stringency must not be too high so as to prevent hybridization of the oligonucleotides to the target sequence. Further, the lower the homology of the oligonucleotide to the target sequence, the lower the stringency of the assay conditions should be, although the stringency must not be too low to allow hybridization to non-specific nucleic acid sequences.

Oligonucleotides of the invention may be prepared by any of a variety of methods (see, for example, J. Sambrook et al., "Molecular Cloning: A Laboratory Manual", 1989, 2.sup.nd Ed., Cold Spring Harbour Laboratory Press: New York, N.Y.; "PCR Protocols: A Guide to Methods and Applications", 1990, M. A. Innis (Ed.), Academic Press: New York, N.Y.; P. Tijssen "Hybridization with Nucleic Acid Probes--Laboratory Techniques in Biochemistry and Molecular Biology (Parts I and II)", 1993, Elsevier Science; "PCR Strategies", 1995, M. A. Innis (Ed.), Academic Press: New York, N.Y.; and "Short Protocols in Molecular Biology", 2002, F. M. Ausubel (Ed.), 5.sup.th Ed., John Wiley & Sons: Secaucus, N.J.). For example, oligonucleotides may be prepared using any of a variety of chemical techniques well-known in the art, including, for example, chemical synthesis and polymerization based on a template as described, for example, in S. A. Narang et al., Meth. Enzymol. 1979, 68: 90-98; E. L. Brown et al., Meth. Enzymol. 1979, 68: 109-151; E. S. Belousov et al., Nucleic Acids Res. 1997, 25: 3440-3444; D. Guschin et al., Anal. Biochem. 1997, 250: 203-211; M. J. Blommers et al., Biochemistry, 1994, 33: 7886-7896; and K. Frenkel et al., Free Radic. Biol. Med. 1995, 19: 373-380; and U.S. Pat. No. 4,458,066.

For example, oligonucleotides may be prepared using an automated, solid-phase procedure based on the phosphoramidite approach. In such a method, each nucleotide is individually added to the 5'-end of the growing oligonucleotide chain, which is attached at the 3'-end to a solid support. The added nucleotides are in the form of trivalent 3'-phosphoramidites that are protected from polymerization by a dimethoxytriyl (or DMT) group at the 5'-position. After base-induced phosphoramidite coupling, mild oxidation to give a pentavalent phosphotriester intermediate and DMT removal provides a new site for oligonucleotide elongation. The oligonucleotides are then cleaved off the solid support, and the phosphodiester and exocyclic amino groups are deprotected with ammonium hydroxide. These syntheses may be performed on oligo synthesizers such as those commercially available from Perkin Elmer/Applied Biosystems, Inc. (Foster City, Calif.), DuPont (Wilmington, Del.) or Milligen (Bedford, Mass.). Alternatively, oligonucleotides can be custom made and ordered from a variety of commercial sources well-known in the art, including, for example, the Midland Certified Reagent Company (Midland, Tex.), ExpressGen, Inc. (Chicago, Ill.), Operon Technologies, Inc. (Huntsville, Ala.), and many others.

Purification of the oligonucleotides of the invention, where necessary or desirable, may be carried out by any of a variety of methods well-known in the art. Purification of oligonucleotides is typically performed either by native acrylamide gel electrophoresis, by anionexchange HPLC as described, for example, by J. D. Pearson and F. E. Regnier (J. Chrom., 1983, 255: 137-149) or by reverse phase HPLC (G. D. McFarland and P. N. Borer, Nucleic Acids Res., 1979, 7: 1067-1080).

The sequence of oligonucleotides can be verified using any suitable sequencing method including, but not limited to, chemical degradation (A. M. Maxam and W. Gilbert, Methods of Enzymology, 1980, 65: 499-560), matrix-assisted laser desorption ionization time-of-flight (MALDI-TOF) mass spectrometry (U. Pieles et al., Nucleic Acids Res., 1993, 21: 3191-3196), mass spectrometry following a combination of alkaline phosphatase and exonuclease digestions (H. Wu and H. Aboleneen, Anal. Biochem., 2001, 290: 347-352), and the like.

In certain embodiments, the detection probes or amplification primers or both probes and primers are labeled with a detectable agent (i.e. detectable moiety or label) before being used in amplification/detection assays. In certain embodiments, the detection probes are labeled with a detectable agent. Preferably, a detectable agent is selected such that it generates a signal which can be measured and whose intensity is related (e.g., proportional) to the amount of amplification products in the sample being analyzed.

The association between the oligonucleotide and detectable agent can be covalent or noncovalent. Labeled detection probes can be prepared by incorporation of or conjugation to a detectable moiety. Labels can be attached directly to the nucleic acid sequence or indirectly (e.g., through a linker). Linkers or spacer arms of various lengths are known in the art and are commercially available, and can be selected to reduce steric hindrance, or to confer other useful or desired properties to the resulting labeled molecules (see, for example, E. S. Mansfield et al., Mol. Cell. Probes, 1995, 9: 145-156).

Methods for labeling nucleic acid molecules are well-known in the art. For a review of labeling protocols, label detection techniques, and recent developments in the field, see, for example, L. J. Kricka, Ann. Clin. Biochem. 2002, 39: 114-129; R. P. van Gijlswijk et al., Expert Rev. Mol. Diagn. 2001, 1: 81-91; and S. Joos et al., J. Biotechnol. 1994, 35: 135-153. Standard nucleic acid labeling methods include: incorporation of radioactive agents, direct attachments of fluorescent dyes (L. M. Smith et al., Nucl. Acids Res., 1985, 13: 2399-2412) or of enzymes (B. A. Connoly and O. Rider, Nucl. Acids. Res., 1985, 13: 4485-4502); chemical modifications of nucleic acid molecules making them detectable immunochemically or by other affinity reactions (T. R. Broker et al., Nucl. Acids Res. 1978, 5: 363-384; E. A. Bayer et al., Methods of Biochem. Analysis, 1980, 26: 1-45; R. Langer et al., Proc. Natl. Acad. Sci. USA, 1981, 78: 6633-6637; R. W. Richardson et al., Nucl. Acids Res. 1983, 11: 6167-6184; D. J. Brigati et al., Virol. 1983, 126: 32-50; P. Tchen et al., Proc. Natl. Acad. Sci. USA, 1984, 81: 3466-3470; J. E. Landegent et al., Exp. Cell Res. 1984, 15: 61-72; and A. H. Hopman et al., Exp. Cell Res. 1987, 169: 357-368); and enzyme-mediated labeling methods, such as random priming, nick translation, PCR and tailing with terminal transferase (for a review on enzymatic labeling, see, for example, J. Temsamani and S. Agrawal, Mol. Biotechnol. 1996, 5: 223-232). More recently developed nucleic acid labeling systems include, but are not limited to: ULS (Universal Linkage System), which is based on the reaction of mono-reactive cisplatin derivatives with the N7 position of guanine moieties in DNA (R. J. Heetebrij et al., Cytogenet. Cell. Genet. 1999, 87: 47-52), psoralen-biotin, which intercalates into nucleic acids and upon UV irradiation becomes covalently bonded to the nucleotide bases (C. Levenson et al., Methods Enzymol. 1990, 184: 577-583; and C. Pfannschmidt et al., Nucleic Acids Res. 1996, 24: 1702-1709), photoreactive azido derivatives (C. Neves et al., Bioconjugate Chem. 2000, 11: 51-55), and DNA alkylating agents (M. G. Sebestyen et al., Nat. Biotechnol. 1998, 16: 568-576).

In certain embodiments, the inventive detection probes are fluorescently labeled. Numerous known fluorescent labeling moieties of a wide variety of chemical structures and physical characteristics are suitable for use in the practice of this invention. Suitable fluorescent dyes include, but are not limited to, fluorescein and fluorescein dyes (e.g., fluorescein isothiocyanine or FITC, naphthofluorescein, 4',5'-dichloro-2',7'-dimethoxy-fluorescein, 6 carboxyfluorescein or FAM), carbocyanine, merocyanine, styryl dyes, oxonol dyes, phycoerythrin, erythrosin, eosin, rhodamine dyes (e.g., carboxytetramethylrhodamine or TAMRA, carboxyrhodamine 6G, carboxy-X-rhodamine (ROX), lissamine rhodamine B, rhodamine 6G, rhodamine Green, rhodamine Red, tetramethylrhodamine or TMR), coumarin and coumarin dyes (e.g., methoxycoumarin, dialkylaminocoumarin, hydroxycoumarin and aminomethylcoumarin or AMCA), Oregon Green Dyes (e.g., Oregon Green 488, Oregon Green 500, Oregon Green 514), Texas Red, Texas Red-X, Spectrum Red^{™}, Spectrum Green^{™}, cyanine dyes (e.g., Cy-3^{™}, Cy-5^{™}, Cy-3.5^{™}, Cy-5.5 ^{™}), Alexa Fluor dyes (e.g., Alexa Fluor 350, Alexa Fluor 488, Alexa Fluor 532, Alexa Fluor 546, Alexa Fluor 568, Alexa Fluor 594, Alexa Fluor 633, Alexa Fluor 660 and Alexa Fluor 680), BODIPY dyes (e.g., BODIPY FL, BODIPY R6G, BODIPY TMR, BODIPY TR, BODIPY 530/550, BODIPY 558/568, BODIPY 564/570, BODIPY 576/589, BODIPY 581/591, BODIPY 630/650, BODIPY 650/665), IRDyes (e.g., IRD40, IRD 700, IRD 800), and the like. For more examples of suitable fluorescent dyes and methods for linking or incorporating fluorescent dyes to nucleic acid molecules see, for example, "The Handbook of Fluorescent Probes and Research Products", 9th Ed., Molecular Probes, Inc., Eugene, Oreg. Fluorescent dyes as well as labeling kits are commercially available from, for example, Amersham Biosciences, Inc. (Piscataway, N.J.), Molecular Probes Inc. (Eugene, Oreg.), and New England Biolabs Inc. (Beverly, Mass.).Another contemplated method of analyzing the methylation status of the sequences is by analysis of the DNA following exposure to methylation-sensitive restriction enzymes - see for example US Application Nos. 20130084571 and 20120003634, the contents of which are incorporated herein.

Exemplary probes for identifying cardiac cells are set forth in SEQ ID NOs: 118 and 119.

Exemplary probes for detecting colon cells are set forth in SEQ ID NOs: 186 (TTGGGGTTTGGGATGTGAGG) and 121 (AAAACCAACCTTATCCCACCTCA).

Exemplary probes for detecting liver cells are set forth in SEQ ID NOs: 122 (TATTGATGGGGTTTTTGATGTTTTAG), 123 (ATACCACCTTCACCCACATCAA). A single probe that can be used to detect liver cells is set forth in SEQ ID NO: 124 (TTAGGTGATTTGTGATTTGTGTATTTATAG).

In one embodiment, the probes that are used are Taqman^{TM} probes.

Taqman^{TM} probes comprise a detectable moiety (e.g. fluorophore) covalently attached to the 5'-end of the oligonucleotide probe and a quencher at the 3'-end. Several different fluorophores (e.g. 6-carboxyfluorescein, acronym: FAM, or tetrachlorofluorescein, acronym: TET) and quenchers (e.g. tetramethylrhodamine, acronym: TAMRA) are available. The quencher molecule quenches the fluorescence emitted by the fluorophore when excited by the cycler's light source via FRET (Förster Resonance Energy Transfer). As long as the fluorophore and the quencher are in proximity, quenching inhibits any fluorescence signals.

Taqman^{TM} probes are designed such that they anneal within a DNA region amplified by a specific set of primers. As the Taq polymerase extends the primer and synthesizes the nascent strand, the 5' to 3' exonuclease activity of the Taq polymerase degrades the probe that has annealed to the template. Degradation of the probe releases the detectable moiety from it and breaks the close proximity to the quencher, thus relieving the quenching effect and allowing for detection of the detectable moiety (e.g. it allow for fluorescence of the fluorophore). Hence, the amount of detectable moiety is directly proportional to the amount of DNA template present in the PCR.

Exemplary targets that may be analyzed according to this aspect of the present invention are provided in US Patent Application No. 20170121767, the contents of which are incorporated herein by reference.

Other exemplary targets that may be analyzed are comprised in any of the sequences set forth in SEQ ID Nos: 2-117 or 128-184. According to a particular embodiment, the target sequence which is analyzed comprises the nucleotides CG which are at position 250 and 251 of each of these sequences.

According to another embodiment, at least one of the methylation sites of the signature are the nucleotides CG which are at position 250 and 251 of each of these sequences.

### Kits

Any of the components described herein may be comprised in a kit. In a non-limiting example the kit comprises
(i) a set of primers that are capable of amplifying the forward strand of the double-stranded DNA molecule and not the reverse strand of the double-stranded DNA molecule;
(ii) a set of primers that are capable of amplifying the reverse strand of the double-stranded DNA molecule and not the forward strand of the double-stranded DNA molecule.

Detectable moieties, quenching moieties and probes have been described herein above.

Additional components that may be included in any of the above described kits include at least one of the following components: a droplet forming oil, bisulfite (and other reagents necessary for the bisulfite reaction), reagents for purification of DNA, MgCl₂. The kit may also comprise reaction components for sequencing the amplified or non-amplified sequences.

The kits may also comprise DNA sequences which serve as controls. Thus, for example, the kit may comprise a DNA having the same sequence as the amplified sequence derived from a healthy subject (to serve as a negative control) and/or a DNA having the same sequence as the amplified sequence derived from a subject known to have the disease which is being investigated (to serve as a positive control).

In addition, the kits may comprise known quantities of DNA such that calibration and quantification of the test DNA may be carried out.

The containers of the kits will generally include at least one vial, test tube, flask, bottle, syringe or other containers, into which a component may be placed, and preferably, suitably aliquoted. Where there is more than one component in the kit, the kit also will generally contain a second, third or other additional container into which the additional components may be separately placed. However, various combinations of components may be comprised in a container.

When the components of the kit are provided in one or more liquid solutions, the liquid solution can be an aqueous solution. However, the components of the kit may be provided as dried powder(s). When reagents and/or components are provided as a dry powder, the powder can be reconstituted by the addition of a suitable solvent.

A kit will preferably include instructions for employing, the kit components as well the use of any other reagent not included in the kit. Instructions may include variations that can be implemented.

### Diagnostics

It will be appreciated that analysis of the methylation status according to methods described herein allows for the accurate determination of cellular/tissue source of a DNA molecule, even when the majority of the DNA of the sample is derived from a different cellular source. The present inventors have shown that they are able to determine the cellular source of a particular DNA even when its contribution to the total amount of DNA in the population is less than 1:1000, less than 1:5,000, 1:10,000 or even 1:100,000.

Pathological and disease conditions that involve cell death cause the release of degraded DNA from dying cells into body fluids (blood, plasma, urine, cerebrospinal fluid). Thus, the methods described herein may be used to analyze the amount of cell death of a particular cell population in those body fluids. The amount of cell death of a particular cell population can then be used to diagnose a particular pathological state (e.g. disease) or condition (e.g. trauma).

It will be appreciated that death of a particular cell type may be associated with a pathological state - e.g. disease or trauma.

The monitoring of the death of a particular cell type may also be used for monitoring the efficiency of a therapeutic regime expected to effect cell death of a specific cell type.

The determination of death of a specific cell type may also be used in the clinical or scientific study of various mechanism of healthy or diseased subjects.

Thus, for example measurement of pancreatic beta cell death is important in cases of diabetes, hyperinsulinism and islet cell tumors, and in order to monitor beta cell survival after islet transplantation, determining the efficacy of various treatment regimes used to protect beta cells from death, and determining the efficacy of treatments aimed at causing islet cell death in islet cell tumors. Similarly, the method allows the identification and quantification of DNA derived from dead kidney cells (indicative of kidney failure), dead neurons (indicative of traumatic brain injury, amyotrophic lateral sclerosis (ALS), stroke, Alzheimer's disease, Parkinson's disease or brain tumors, with or without treatment); dead pancreatic acinar cells (indicative of pancreatic cancer or pancreatitis); dead lung cells (indicative of lung pathologies including lung cancer); dead adipocytes (indicative of altered fat turnover), dead hepatocytes (indicative of liver failure, liver toxicity or liver cancer) dead cardiomyocytes (indicative of cardiac disease, or graft failure in the case of cardiac transplantation), dead skeletal muscle cells (indicative of muscle injury and myopathies), dead oligodendrocytes (indicative of relapsing multiple sclerosis, white matter damage in amyotrophic lateral sclerosis, or glioblastoma), dead colon cells is indicative of colorectal cancer.

As used herein, the term "diagnosing" refers to determining the presence of a disease, classifying a disease, determining a severity of the disease (grade or stage), monitoring disease progression and response to therapy, forecasting an outcome of the disease and/or prospects of recovery.

The method comprises quantifying the amount of cell-free DNA which is comprised in a fluid sample (e.g. a blood sample or serum sample) of the subject which is derived from a cell type or tissue. When the amount of cell free DNA derived from the cell type or tissue is above a predetermined level, it is indicative that there is a predetermined level of cell death. When the level of cell death is above a predetermined level, it is indicative that the subject has the disease or pathological state. Determining the predetermined level may be carried out by analyzing the amount of cell-free DNA present in a sample derived from a subject known not to have the disease/pathological state. If the level of the cell-free DNA derived from a cell type or tissue associated with the disease in the test sample is statistically significantly higher (e.g. at least two fold, at least three fold, or at least 4 fold) than the level of cell-free DNA derived from the same cell type or tissue in the sample obtained from the healthy (non-diseased subject), it is indicative that the subject has the disease. Alternatively, or additionally, determining the predetermined level may be carried out by analyzing the amount of cell-free DNA present in a sample derived from a subject known to have the disease. If the level of the cell-free DNA derived from a cell type or tissue associated with the disease in the test sample is statistically significantly similar to the level of the cell-free DNA derived from a cell type of tissue associated with the disease in the sample obtained from the diseased subject, it is indicative that the subject has the disease.

The severity of disease may be determined by quantifying the amount of DNA molecules having the specific methylation pattern of a cell population associated with the disease. Quantifying the amount of DNA molecules having the specific methylation pattern of a target tissue may be achieved using a calibration curve produced by using known and varying numbers of cells from the target tissue.

According to one embodiment, the method comprises determining the ratio of the amount of cell free DNA derived from a cell of interest in the sample: amount of overall cell free DNA.

According to still another embodiment, the method comprises determining the ratio of the amount of cell free DNA derived from a cell of interest in the sample: amount of cell free DNA derived from a second cell of interest.

The methods described herein may also be used to determine the efficacy of a therapeutic agent or treatment, wherein when the amount of DNA associated with a cell population associated with the disease is decreased following administration of the therapeutic agent, it is indicative that the agent or treatment is therapeutic.

According to some embodiments of the invention, screening of the subject for a specific disease is followed by substantiation of the screen results using gold standard methods.

The method can also be used to predict prognosis of the subject with the disease.

According to some embodiments of the invention, the method further comprising informing the subject of the predicted disease and/or the predicted prognosis of the subject.

As used herein the phrase "informing the subject" refers to advising the subject that based on the cfDNA levels, the subject should seek a suitable treatment regimen.

Once the cfDNA level is determined, the results can be recorded in the subject's medical file, which may assist in selecting a treatment regimen and/or determining prognosis of the subject.

According to some embodiments of the invention, the method further comprising recording the cf DNA levels of the subject in the subject's medical file.

As mentioned, the prediction can be used to select the treatment regimen of a subject and thereby treat the subject in need thereof.

It is expected that during the life of a patent maturing from this application many relevant sequencing technologies will be developed (including those that will be able to determine methylation status, without bisulfite treatment) and the scope of the term sequencing is intended to include all such new technologies *a priori*.

As used herein the term "about" refers to ± 10 %
The terms "comprises", "comprising", "includes", "including", "having" and their conjugates mean "including but not limited to".

The term "consisting of" means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

It is understood that any Sequence Identification Number (SEQ ID NO) disclosed in the instant application can refer to either a DNA sequence or a RNA sequence, depending on the context where that SEQ ID NO is mentioned, even if that SEQ ID NO is expressed only in a DNA sequence format or a RNA sequence format. For example, SEQ ID NO: XXX is expressed in a DNA sequence format (*e.g.*, reciting T for thymine), but it can refer to either a DNA sequence that corresponds to an XXX nucleic acid sequence, or the RNA sequence of an RNA molecule nucleic acid sequence. Similarly, though some sequences are expressed in a RNA sequence format (*e.g.*, reciting U for uracil), depending on the actual type of molecule being described, it can refer to either the sequence of a RNA molecule comprising a dsRNA, or the sequence of a DNA molecule that corresponds to the RNA sequence shown. In any event, both DNA and RNA molecules having the sequences disclosed with any substitutes are envisioned.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Various embodiments and aspects of the present invention as delineated hereinabove and as claimed in the claims section below find experimental support in the following examples.

### EXAMPLES

Reference is now made to the following examples, which together with the above descriptions illustrate some embodiments of the invention in a non limiting fashion.
Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Culture of Animal Cells - A Manual of Basic Technique" by Freshney, Wiley-Liss, N. Y. (1994), Third Edition; "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996); all of which are incorporated by reference as if fully set forth herein. Other general references are provided throughout this document. The procedures therein are believed to be well known in the art and are provided for the convenience of the reader. All the information contained therein is incorporated herein by reference.

### EXAMPLE 1

### MATERIALS AND METHODS

***Clinical samples:*** Cardiac biomarkers used were troponin T and CPK.

***Identification of cardiac methylation markers:*** Tissue-specific DNA methylation markers were selected after a comparison of publically available DNA methylation datasets generated by whole-genome bisulfite sequencing (Roadmap Epigenomics). The fragment of FAM101A used as a cariomyocyte-specific marker is located in chromosome 12, coordinates124692462-124692551.

***cfDNA analysis:*** Blood samples were collected in EDTA tubes, and centrifuged within 2 hours to separate plasma from peripheral blood cells: first at 1500g for 10 min, and then at 3000g for 10 min to remove any remaining cells. Plasma was then stored at -80°C.

cfDNA was extracted using the QIAsymphony SP instrument and its dedicated QIAsymphony Circulating DNA Kit (Qiagen) according to the manufacturer's instructions. DNA concentration was measured using the Qubit^{™} dsDNA HS Assay Kit.

cfDNA was treated with bisulfite using a kit (Zymo Research), and PCR amplified with primers specific for bisulfite-treated DNA but independent of methylation status at the monitored CpG sites. Primers were bar-coded, allowing the mixing of samples from different individuals when sequencing PCR products using MiSeq or NextSeq (Illumina). Sequenced reads were separated by barcode, aligned to the target sequence, and analyzed using custom scripts written and implemented in R. Reads were quality filtered based on Illumina quality scores, and identified by having at least 80% similarity to target sequences and containing all the expected CpGs in the sequence. CpGs were considered methylated if "CG" was read and were considered unmethylated if "TG" was read.

***Digital Droplet PCR:*** A procedure was established for digital droplet PCR, in which bisulfite-treated cfDNA is amplified using a methylation-sensitive Taqman^{TM} probe.

The limited length of probes (up to 30 bp) dictated that they could cover only 2 or 3 informative CpG sites in the FAM101A locus, predicting a relatively high frequency of "noise" (positive droplets) in DNA from non-cardiac tissue. In the sequencing-based assay, this problem was addressed by documenting the methylation status of multiple adjacent cytosines (FIGs. 1A-E), which greatly increased specificity.

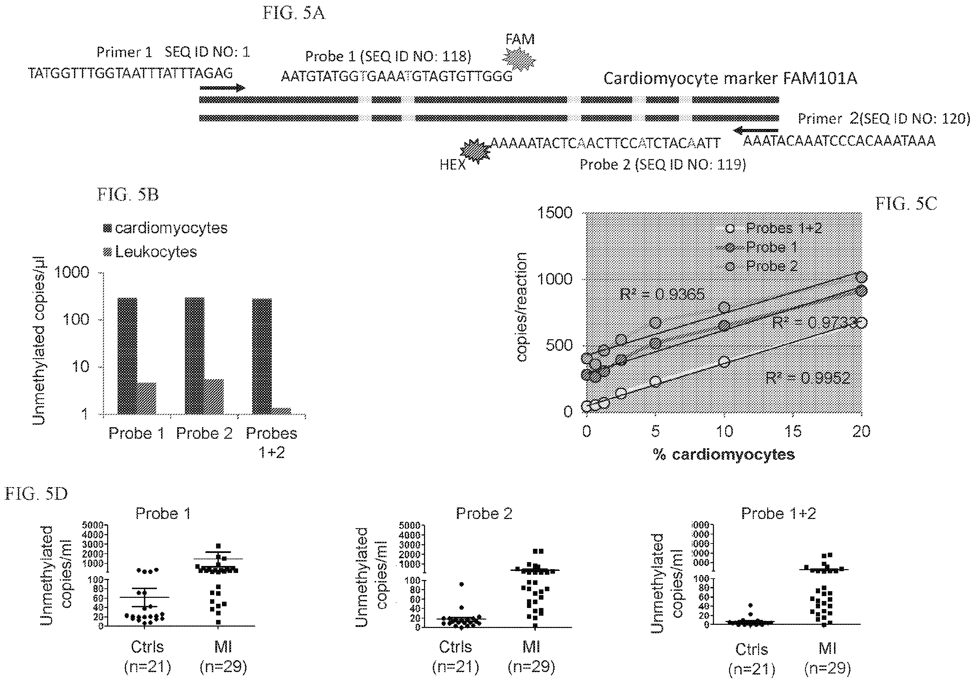

To implement this concept in the ddPCR platform, two Taqman^{TM} probes were designed, each recognizing lack of methylation in a different cluster of cytosines (one containing 2 CpG sites and one containing 3 CpG sites) within the same amplified 100bp fragment from the FAM101A locus (FIG. 5A). Each probe was labeled with a different fluorophore, such that droplets could be identified in which both probes found a target. Such droplets would be interpreted as containing a FAM101A cfDNA fragment in which all 5 targeted cytosines were demethylated. This would provide ddPCR with the improved specificity afforded by interrogating multiple cytosines on the same DNA molecule.

For the analysis of 5 cytosines, located adjacent to the FAM101A locus, the following primers were used: 5'-TATGGTTTGGTAATTTATTTAGAG-3' (SEQ ID NO: 1; forward) and 5'-AAATACAAATCCCACAAATAAA-3' (SEQ ID NO: 120; reverse) in combination with probes that detected lack of methylation on 3 and 2 cytosines respectively: 5'-AATGTATGGTGAAATGTAGTGTTGGG -3' (SEQ ID NO: 118; FAM-forward probe) and 5'- AAAAATACTCAACTTCCATCTACAATT -3' (SEQ ID NO: 119, HEX-reverse probe).

Assay design is shown in FIG. 5A. Each 20-µL volume reaction mix consisted of ddPCR^{™} Supermix for Probes (No dUTP) (Bio-Rad), 900nM primer, 250nM probe, and 2 µL of sample. The mixture and droplet generation oil were loaded onto a droplet generator (Bio-Rad). Droplets were transferred to a 96-well PCR plate and sealed. The PCR was run on a thermal cycler as follows: 10 minutes of activation at 95°C, 47 cycles of a 2 step amplification protocol (30 s at 94°C denaturation and 60 s at 53.7 °C), and a 10-minute inactivation step at 98°C. The PCR plate was transferred to a QX100Droplet Reader (Bio-Rad), and products were analyzed with QuantaSoft (Bio-Rad) analysis software. Discrimination between droplets that contained the target (positives) and those which did not (negatives) was achieved by applying a fluorescence amplitude threshold based on the amplitude of reads from the negative template control.

### RESULTS

### Identification of cardiomyocyte methylation markers

To define genomic loci that are methylated in a cardiac-specific manner, the methylomes of human heart chambers (right atrium, left and right ventricle) were compared with the methylomes of 23 other human tissues, all publicly available¹². Several differentially methylated loci were identified and a cluster of cytosines adjacent to the FAM101A locus was selected for further analysis (FIGs. 1A and 1B). PCR was used to amplify a 90bp fragment around this cluster after bisulfite conversion of unmethylated cytosines, and the PCR product was sequenced to determine the methylation status of all 6 cytosines in the cluster. In purified cardiomyocyte DNA, 89% of the molecules were fully unmethylated, while in non-cardiac tissue <0.2% of molecules were unmethylated; specifically in leukocytes (the main contributor to cfDNA), <0.006% of molecules were unmethylated (FIGs. 1C and 6A-C). Thus, interrogating all CpGs simultaneously, the ratio of demethylated molecules in heart:blood DNA was 89:0.006 giving a signal to noise ratio of 15,000.

To determine the linearity and sensitivity of the assay, leukocyte DNA was spiked with increasing amounts of cardiac DNA. The fraction of cardiac DNA in the mixture was assessed using PCR amplification and massively parallel sequencing. The assay was able to correctly determine the fraction of cardiac DNA, even when it was only 0.5% of the DNA in the mixture (FIG. 1D).

Following bisulfite treatment, DNA becomes single stranded. Therefore, each strand can be considered an independent biomarker. To test this idea, the present inventors designed primers against the antisense strand of FAM101A post-bisulfite conversion. As expected, the sense and antisense templates showed a similar sensitivity and specificity (FIGs. 1B-E and 6A-C). It was reasoned that by testing both strands in a given sample, both sensitivity and specificity of the assay will increase. For this reason further analysis of clinical samples was performed using both sense and antisense specific primer sets.

### Plasma levels of cardiomyocyte DNA in healthy individuals

The sense and antisense FAM101A markers were used to assess the concentration of cardiac cfDNA in the plasma of donors. cfDNA was extracted from plasma and treated with bisulfite. PCR and sequencing were performed, typically using material from 0.5ml of plasma. The fraction of PCR products carrying the cardiac-specific methylation pattern was multiplied by the total concentration of cfDNA, to obtain an estimation of cardiac cfDNA content in plasma.

Healthy adult plasma from 83 healthy donors was tested and zero copies of cardiac cfDNA were detected in 73 of them (FIG. 2A). In ten individuals, 1-20 copies/ml cardiac cfDNA was found. This low level of a signal likely reflects the low rate of cardiomyocyte death in healthy adults¹³. The mean plus 2 standard deviations of the control group was 10 copies/ml, and this was thus defined as the cutoff level for a positive signal.

***Plasma levels of cardiomyocyte DNA after myocardial infarction:*** As a positive control where high levels of cardiac cfDNA are expected, plasma from donors with myocardial infarction (MI) were used. Samples from individuals that presented with chest pain, before and after they underwent angioplasty were used. The levels of cardiac cfDNA as well as troponin and CPK were assessed. MI patients showed dramatically higher levels of cardiac cfDNA than healthy controls (FIG. 2A and FIGs. 7A-F and 8A-B). To assess assay performance in discriminating healthy from MI plasma a Receiver Operator Characteristic (ROC) curve was plotted. The area under the curve (AUC) was 0.9345, indicating high sensitivity and specificity (FIG. 2B). The present inventors also compared cardiac cfDNA to standard cardiac damage markers CPK and troponin. Compared with healthy controls, cardiac cfDNA was significantly higher in MI patients that had CPK just above normal (<200), and was even higher in patients with high CPK (>200) (FIG. 2C). Similarly, cardiac cfDNA was higher than normal in plasma samples that had either low or high levels of troponin (FIG. 2D and FIGs. 7A-F). Among the 6 samples that had troponin levels above baseline but <0.03, there was no more cfDNA than in healthy controls (FIG. 2D).

A comparison of troponin levels to cardiac cfDNA in 57 samples from MI patients yielded Spearman correlation value of 0.7975 and p<0.0001 (FIG. 2E). When plotting cardiac cfDNA vs troponin and marking on each axis the threshold of a positive signal, it was found that 79% of the MI samples were positive for both troponin and cardiac cfDNA, and 7% were negative for both. 11% were positive only for troponin, and 4% were positive only for cardiac cfDNA (FIG. 2F). Importantly, total levels of cfDNA in MI did not correlate with troponin or CPK, nor with the percentage of cardiac cfDNA (FIGs. 7A-F). This reflects that fact that total cfDNA integrates all recent cell death events, including contributions from tissues that mask the cardiac signal. Thus, it is essential to calculate the specific contribution of the heart to cfDNA in order to assess cardiac damage. The sense and antisense markers correlated well in the MI plasma samples (FIGs. 7A-F).

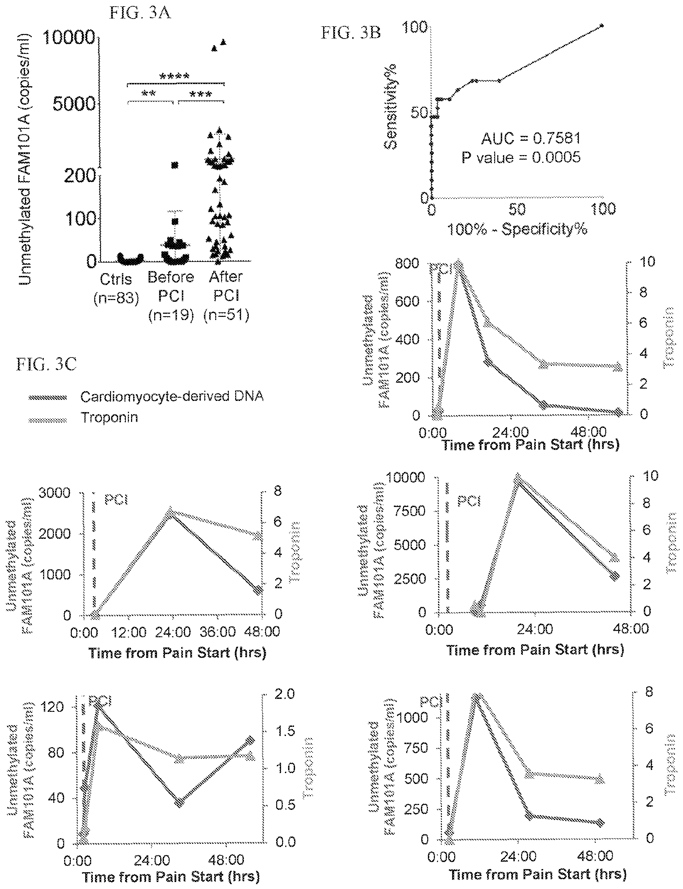

Finally, the present inventors examined the dynamics of cardiac cfDNA before and after angioplasty (Percutaneous Coronary Intervention, PCI). PCI causes the release of trapped cardiac material into blood, hence increased levels of troponin post PCI are typical of successful reperfusion. Cardiac cfDNA levels increased dramatically in most patients after PCI (FIG. 3A and supplemental FIGs. 8A-B), further supporting authenticity of the signal. A more detailed time course on a smaller group of patients revealed that cardiac cfDNA levels rose quickly after PCI and returned to baseline after 1-2 days, showing similar kinetics to troponin and CPK (FIG. 3B and supplemental FIGs. 8A-B). Importantly, the cardiac cfDNA signal was sufficient to distinguish people with MI prior to intervention (0-2 hours after onset of chest pain) from healthy individuals (AUC=0.7616, p=0.0044, FIG. 3C).

It can be concluded that measurements of cardiac cfDNA captures cardiomyocyte cell death associated with myocardial infarction, and that the cardiac cfDNA assay can in principle identify MI before intervention.

### Cardiomyocyte cfDNA in patients with sepsis

Some septic patients have elevated levels of troponin and CPK¹⁴, although they do not show clinical evidence of cardiac damage ^{15, 16}. The biological significance of this observation is disputed, since high troponin could represent either cardiomyocyte death, or alternatively transient stress absent of cell death. Since renal dysfunction is common in sepsis, the elevation in circulating troponin may also result from slower clearance, rather than faster release of troponin¹⁷. Since cfDNA is a stronger marker of cell death and is cleared by the liver¹⁸, it was reasoned that measurements of cardiac cfDNA can be informative in this setting.

The present inventors determined the levels of cardiac cfDNA in a cohort of 100 patients with sepsis, for which 201 plasma samples were available. Cardiac cfDNA was assessed blindly, and values were correlated to other biomarkers and to clinical parameters.

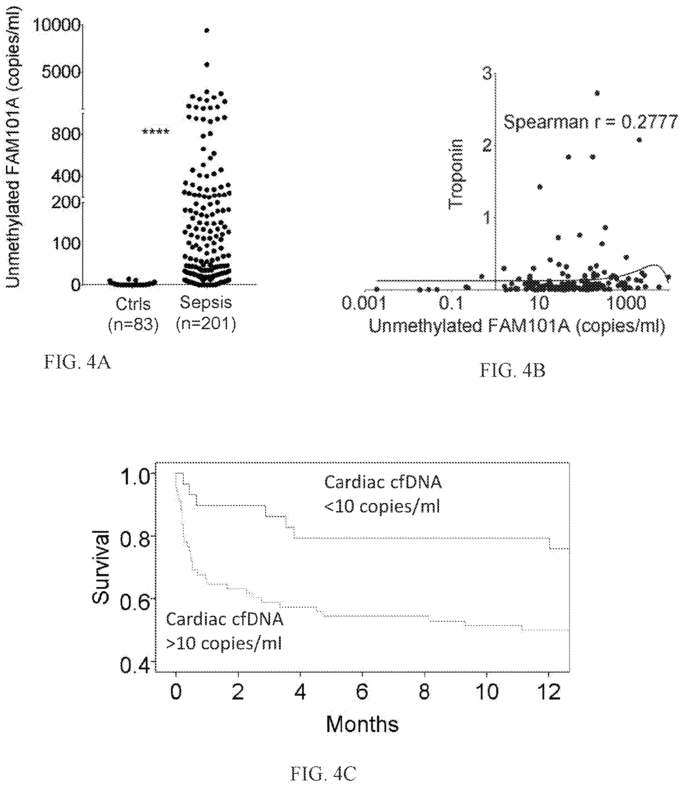

Septic patients had high levels of total cfDNA, reflective of broad tissue damage (Figures 9A-C), as reported¹⁹. Strikingly, many patients had high levels of cardiac cfDNA, similar in magnitude to the acute setting of MI (FIG. 4A). These findings argue strongly that in many septic patients, massive cardiomyocyte death occurs. The sense and antisense markers of FAM101A correlated well, supporting specificity of the signal (Figures 9A-C). Cardiac cfDNA and troponin levels did not correlate in the sepsis, unlike the situation in MI (FIG. 4B). This is not surprising, given the chronic nature of tissue damage in sepsis, which is expected to involve a major contribution of clearance rates on the actual measurements of biomarkers. A dramatic elevation of cardiac cfDNA was seen also in septic patients with normal renal function (data not shown), supporting the idea that cardiac cfDNA reflects cell death and not altered clearance rate.

The present inventors attempted to correlate the levels of cardiac cfDNA with clinical parameters recorded for the sepsis patients. The presence of cardiac cfDNA was strongly correlated with short-term mortality (FIG. 4C). When excluding cases with sepsis in the background of advanced cancer, patients with cardiac cfDNA were 4 times more likely to die within 90 days of hospitalization than patients with no cardiac cfDNA. The correlation was stronger than the correlation between troponin and mortality or between total cfDNA and mortality, but weaker than the correlation between age and mortality. These findings indicate that cardiac function is a central determinant of patient survival under sepsis, and that cardiac cfDNA can be used as a prognostic biomarker in sepsis.

### A modified digital droplet PCR procedure for measurement of cardiac cfDNA

In order to translate analysis of cfDNA to a simpler and faster PCR format, the present inventors established a procedure using digital droplet PCR (ddPCR) to accurately count the number of molecules carrying the cardiac methylation signature at the FAM101A locus. They designed the assay to simultaneously interrogate 5 CpGs in the locus using two fluorescent probes, each capturing distinct 2 or 3 unmethylated cytosines (FIG. 5A), leveraging the increased specificity attributed to regional methylation status⁹.

ddPCR analysis of cardiomyocyte and leukocyte DNA revealed that each probe alone was able to discriminate between DNA from the two sources, with a signal to noise ratio of 50 to 58. However, when only droplets positive for both probes were scored, the cardiomyocyte:leukocyte signal ratio increased to 258, affording a 5 fold increase in specificity (FIG. 5B). ddPCR on cardiac DNA spiked into leukocyte DNA gave a signal that increased linearly with the amount of cardiac DNA; scoring only dual-labeled probes gave a lower baseline signal than scoring individual probes, better reflecting cardiomyocyte contribution to the mixture (FIG. 5C).

Finally, the ddPCR assay was tested on plasma samples. ddPCR revealed a clear signal in the plasma of MI patients and was able to distinguish well between controls and patients. A lower baseline signal was observed in healthy individuals when scoring only dual-labeled probes, indicating increased specificity (FIG. 5D). It can be concluded that the ddPCR assay for cardiac cfDNA provides a rapid and simple alternative to sequencing-based assays.

### EXAMPLE 2

### List of additional identified targets

A list of identified targets is provided in Table 1 and 2 herein below. The methylation signature of the targets can be used to identify a cell type of the listed organ. It will be appreciated that the sequences provided are 500 base pairs. Preferably the target sequence (which is amplified which is less than all the 500 base pairs) comprises the nucleotides CG which are at position 250 and 251 of each of these sequences and additional nucleotides up and/or down-stream of this site.

**Table 1**

| ***Organ*/*cell type*** | ***Name of gene*** | ***SEQ ID NO:*** |
|---|---|---|
| Acinar | CPA1 | 2 |
| Acinar | LMF2 | 3 |
| Acinar | NCLN | 4 |
| Acinar | BRF1 | 5 |
| Acinar | FRY | 6 |
| Astrocytes | HDAC4 | 7 |
| Astrocytes | AGAP1 | 8 |
| Astrocytes | AST1 | 9 |
| Astrocytes | PRDM | 10 |
| Astrocytes | FOXP4 | 11 |
| Astrocytes | KIAA | 12 |
| Astrocytes | PRDM2 | 13 |
| Astrocytes | WWOX | 14 |
| B cells | LRP5 | 15 |
| B cells | SORL1 | 16 |
| B cells | TRPV1 | 17 |
| BETA | INSh | 18 |
| BETA | MTG1 | 19 |
| BETA | ZC3H3 | 20 |
| BETA | Leng8 | 21 |
| BETA | Fbxw8 | 22 |
| BETA | Fbxl19 | 23 |
| Blood | Loc1/AGAP2 | 24 |
| Blood | PTPRCAP | 25 |
| BRAIN | MAD1L1 | 26 |
| BRAIN | PTPRN2 | 27 |
| BRAIN | WM1 | 28 |
| BRAIN | MBP | 29 |
| BRAIN | NUMBLE | 30 |
| BRAIN | LRRN3 | 31 |
| BRAIN | cg0978 | 32 |
| BRAIN | ZNF238 | 33 |
| Brain | WB1 | 34 |
| Brain | UBE4B | 35 |
| Breast | KRT19 | 36 |
| Breast | LMX1B | 37 |
| Breast | ZNF296 | 38 |
| CD8 cells | CD8A | 39 |
| CD8 cells | CD8A anti | 40 |
| CD8 cells | CD8B | 41 |
| CD8 cells | CD8B anti | 42 |
| Colon | FGFRL1 | 43 |
| Colon | FAT1 | 44 |
| Colon | col1 | 45 |
| Colon | MG1 | 46 |
| Colon | colnp | 47 |
| Colon | col2np | 48 |
| Colon | ECH1 | 49 |
| Colon | ECH1 | 50 |
| Colon | CNL (my name) | 51 |
| Colon | MAP7D1 | 52 |
| Colon | col3np (my name) | 53 |
| Eosinophils | PCYT1A | 54 |
| Eosinophils | PCYT1A anti | 55 |
| Heart | FAM101A | 56 |
| Heart | FAM101A AS | 57 |
| kidney | cg00256155 | 58 |
| kidney | PAX2 | 59 |
| kidney | cg15767955 | 60 |
| kidney | MCF2L | 61 |
| kidney | HOXC4 | 62 |
| kidney | PAX2 | 63 |
| Liver | ITIH4 | 64 |
| Liver | SEBOX;VTN | 65 |
| Liver | IGF2R | 66 |
| LUNG | SFTP/A1 | 67 |
| LUNG | SFTP/A2 | 68 |
| LUNG | CLDN18 | 69 |
| LUNG | RAB4 | 70 |
| LUNG | CHST | 71 |
| LUNG | SFTPC | 72 |
| Melanocytes | GALNT3-B | 73 |
| Melanocytes | Melano1 | 74 |
| Melanocytes | Melano1 anti | 75 |
| Melanocytes | RNF207-A | 76 |
| Melanocytes | RNF207-A anti | 77 |
| Melanocytes | RNF207-B | 78 |
| Melanocytes | RNF207-B anti | 79 |
| Monocytes | TCF7L2 | 80 |
| Monocytes | MONO1 | 81 |
| Muscle | MAD1L1 | 82 |
| Muscle | TPO | 83 |
| Muscle | TNNI2 | 84 |
| Muscle | TRIM72;PYDC1 | 85 |
| Neuron | ZNF509 | 86 |
| Neuron | ITFG3 | 87 |
| Neuron | CTBP2 | 88 |
| Neuron | SLC38A10 | 89 |
| neutrophils | DENND3 | 90 |
| neutrophils | NEUT1 | 91 |
| NK | RFC2 | 92 |
| Oligodendrocytes | PLEK | 93 |
| Oligodendrocytes | EVI5L | 94 |
| Oligodendrocytes | ZFP57 | 95 |
| Oligodendrocytes | DNAH | 96 |
| Oral cavity | hH&N1 | 97 |
| Oral cavity | CALML3 | 98 |
| Oral cavity | hH&N4 | 99 |
| Pancreas | CUX2 | 100 |
| Pancreas | PAN4 | 101 |
| Pancreas | REG1A | 102 |
| Pancreas | FRY | 103 |
| Pancreas | BRF1 | 104 |
| Pancreas | PRDM16 (not the same as above) | 105 |
| Pancreatic duct | PRDM16 | 106 |
| Small intestine | ST5 | 107 |
| Small intestine | BANP | 108 |
| Small intestine | SS18L1 | 109 |
| T cells | PRKCH | 110 |
| T cells | SPATA13 | 111 |
| Thyroid | ZNF500 | 112 |
| Thyroid | ATP11A | 113 |
| Treg | FOXP3 | 114 |
| Treg | FOXP3 ANTI | 115 |
| Treg | FOXP3 TSDR | 116 |
| Treg | FOXP3 TSDR anti | 117 |

**Table 2**

| Organ/cell type | Name of gene | SEQ ID NO: |
|---|---|---|
| B cells | NAT10 | 129 |
| BETA | GALNTL4 | 130 |
| BETA | cg06081580 | 131 |
| BETA | RGS9 | 132 |
| BETA | DLG5 | 133 |
| BETA | GNAS | 134 |
| BETA | TTC15 | 135 |
| BETA | MAD1L1 | 136 |
| BETA | cg22406334 | 137 |
| BETA | ZDHHC14 | 138 |
| BETA | ZC3H3_a | 139 |
| BETA | SDK1 | 140 |
| BETA | SFRS16 | 141 |
| BETA | PUS3 | 142 |
| BETA | ZC3H3-c | 143 |
| BETA | ACSF3 | 144 |
| BETA | cg19441717 me | 145 |
| White Blood Cells | SNX11 | 146 |
| Cardiomyocytes | Cardio C | 147 |
| Cardiomyocytes | Cardio D | 148 |
| Cardiomyocytes | Cardio E | 149 |
| Cardiomyocytes | Cardio I | 150 |
| Cardiomyocytes | Cardio J | 151 |
| Colon | CNL2 | 152 |
| Colon | CNL | 153 |
| Colon | col3np | 154 |
| Eosinophils | HTT | 155 |
| Eosinophils | ACOT7 | 156 |
| Kidney | ATP11A | 157 |
| Kidney | PAX2-6032 | 158 |
| Kidney | cg00256155 | 159 |
| Kidney | PAX2-818 | 160 |
| Kidney | MCF2L | 161 |
| LUNG | LUAD1 | 162 |
| LUNG | LUAD5 | 163 |
| LUNG | LUSC2 | 164 |
| LUNG | LUSC3 | 165 |
| LUNG | S3-unMe | 166 |
| LUNG | S4-unMe | 167 |
| LUNG | S5-unMe | 168 |
| LUNG | S5-Meth | 169 |
| LUNG | S10-unMe | 170 |
| LUNG | S11-unMe | 171 |
| LUNG | S13-unMe | 172 |
| LUNG | S12-Meth | 173 |
| Melanocytes | RNF207-A | 174 |
| Melanocytes | RNF207-B | 175 |
| Melanocytes | melano1 | 176 |
| Neutrophils | HIPK3 | 177 |
| Oligodendrocyte | NMRAL1 | 178 |
| Oligodendrocyte | TAF8 | 179 |
| Tongue | PIGG | 180 |
| Tongue | MAD1L1 | 181 |
| Tongue | TP73 | 182 |
| Tongue | BAIAP2 | 183 |
| Tongue | HN1L | 184 |
| T regs | FOXP3 TSDR | 185 |

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims.

All publications, patents and patent applications mentioned in this specification are herein incorporated in their entirety by reference into the specification, to the same extent as if each individual publication, patent or patent application was specifically and individually indicated to be incorporated herein by reference. In addition, citation or identification of any reference in this application shall not be construed as an admission that such reference is available as prior art to the present invention. To the extent that section headings are used, they should not be construed as necessarily limiting.

### REFERENCES

1. Hickman, P.E. et al. Cardiac troponin may be released by ischemia alone, without necrosis. Clin Chim Acta 411, 318-323 (2010).
2. Michielsen, E.C., Wodzig, W.K. & Van Dieijen-Visser, M.P. Cardiac troponin T release after prolonged strenuous exercise. Sports medicine 38, 425-435 (2008).
3. Roca, E. et al. The Dynamics of Cardiovascular Biomarkers in non-Elite Marathon Runners. J Cardiovasc Transl Res (2017).
4. Katus, H.A., Remppis, A., Scheffold, T., Diederich, K.W. & Kuebler, W. Intracellular compartmentation of cardiac troponin T and its release kinetics in patients with reperfused and nonreperfused myocardial infarction. Am J Cardiol 67, 1360-1367 (1991).
5. Bianchi, D.W. et al. DNA sequencing versus standard prenatal aneuploidy screening. The New England journal of medicine 370, 799-808 (2014).
6. Dawson, S.J. et al. Analysis of circulating tumor DNA to monitor metastatic breast cancer. The New England journal of medicine 368, 1199-1209 (2013).
7. Snyder, T.M., Khush, K.K., Valantine, H.A. & Quake, S.R. Universal noninvasive detection of solid organ transplant rejection. Proc Natl Acad Sci U S A 108, 6229-6234 (2011).
8. De Vlaminck, I. et al. Circulating cell-free DNA enables noninvasive diagnosis of heart transplant rejection. Sci Transl Med 6, 241ra277 (2014).
9. Lehmann-Werman, R. et al. Identification of tissue-specific cell death using methylation patterns of circulating DNA. Proc Natl Acad Sci U S A 113, E1826-1834 (2016).
10. Sun, K. et al. Plasma DNA tissue mapping by genome-wide methylation sequencing for noninvasive prenatal, cancer, and transplantation assessments. Proc Natl Acad Sci U S A 112, E5503-5512 (2015).
11. Guo, S. et al. Identification of methylation haplotype blocks aids in deconvolution of heterogeneous tissue samples and tumor tissue-of-origin mapping from plasma DNA. Nat Genet 49, 635-642 (2017).
12. Roadmap Epigenomics, C. et al. Integrative analysis of 111 reference human epigenomes. Nature 518, 317-330 (2015).
13. Bergmann, O. et al. Dynamics of Cell Generation and Turnover in the Human Heart. Cell 161, 1566-1575 (2015).
14. Turner, A., Tsamitros, M. & Bellomo, R. Myocardial cell injury in septic shock. Crit Care Med 27, 1775-1780 (1999).
15. Sanfilippo, F. et al. Diastolic dysfunction and mortality in septic patients: a systematic review and meta-analysis. Intensive care medicine 41, 1004-1013 (2015).
16. Hochstadt, A., Meroz, Y. & Landesberg, G. Myocardial dysfunction in severe sepsis and septic shock: more questions than answers? J Cardiothorac Vasc Anesth 25, 526-535 (2011).
17. Friden, V. et al. Clearance of cardiac troponin T with and without kidney function. Clin Biochem (2017).
18. Gauthier, V.J., Tyler, L.N. & Mannik, M. Blood clearance kinetics and liver uptake of mononucleosomes in mice. J Immunol 156, 1151-1156 (1996).
19. Rhodes, A., Wort, S.J., Thomas, H., Collinson, P. & Bennett, E.D. Plasma DNA concentration as a predictor of mortality and sepsis in critically ill patients. Critical care 10, R60 (2006).
20. Shave, R. et al. Exercise-induced cardiac troponin elevation: evidence, mechanisms, and implications. J Am Coll Cardiol 56, 169-176 (2010).
21. Lo, Y.M. et al. Rapid clearance of fetal DNA from maternal plasma. Am J Hum Genet 64, 218-224 (1999).
22. Simpson, J.T. et al. Detecting DNA cytosine methylation using nanopore sequencing. Nature methods 14, 407-410 (2017).
Embodiments of the present invention are listed below as E1 to E34.
E1. A method of determining the methylation status of at least one methylation site of a double-stranded DNA molecule, the method comprising:
   (a) contacting the double-stranded DNA with bisulfite to generate:
      (i) a forward single-stranded DNA molecule of which demethylated cytosines of said single-stranded DNA molecules are converted to uracils and;
      (ii) a reverse single-stranded DNA molecule of which demethylated cytosines of said single-stranded DNA molecules are converted to uracils; and
   (b) determining the methylation status of said at least one methylation site on said forward strand;
   (c) determining the methylation status of said at least one methylation site on said reverse strand, wherein a methylation status of said at least one methylation site on said forward strand and said at least one methylation site on said reverse strand is indicative of the methylation status of said methylation site.
E2. The method of E 1, wherein said double-stranded DNA molecule is no longer than 300 base pairs.
E3. The method of E 1, wherein said double-stranded DNA molecule is no longer than 150 base pairs.
E4. The method of E 1 wherein said double stranded DNA comprises at least two methylation sites per single strand of said double-stranded DNA molecule.
E5. The method of E 4, wherein said at least two methylation sites are not more than 300 bp apart.
E6. The method of E 4, wherein said at least two methylation sites are not more than 150 bp apart.
E7. The method of any one of E 4-6, wherein each strand of said double-stranded DNA comprises at least three methylation sites.
E8. The method of E 7, wherein said at least three methylation sites are not more than 300 bp apart.
E9. The method of E 7, wherein said at least three methylation sites are not more than 150 bp apart.
E10. The method of any one of E 1-9, further comprising amplifying said single-stranded DNA molecule following step (a) and prior to step (b).
E11. The method of E 1, for determining the cell or tissue of origin of the double-stranded DNA molecule.
E12. The method of E 11, wherein said double-stranded DNA molecule is differentially methylated in a cell or tissue of interest.
E13. The method of E 12, wherein said cell of interest is selected from the group consisting of a pancreatic beta cell, a pancreatic exocrine cell, a hepatocyte, a brain cell, a lung cell, a uterus cell, a kidney cell, a breast cell, an adipocyte, a colon cell, a rectum cell, a cardiomyocyte, a skeletal muscle cell, a prostate cell and a thyroid cell.
E14. The method of E 12, wherein said tissue is selected from the group consisting of pancreatic tissue, liver tissue, lung tissue, brain tissue, uterus tissue, renal tissue, breast tissue, fat, colon tissue, rectum tissue, cardiac tissue, skeletal muscle tissue, prostate tissue and thyroid tissue.
E15. The method of E 14, wherein said tissue is cardiac tissue.
E16. The method of E 15, wherein said double-stranded DNA molecule is non-methylated in cells of cardiac tissue and methylated in leukocytes.
E17. The method of E 16, wherein said double-stranded DNA molecule comprises at least a part of the sequence of human chromosome 12, between coordinates124692462-124692551.
E18. The method of E 16, wherein said double-stranded DNA molecule comprises a sequence which is comprised in SEQ ID NOs: 56 or 57.
E19. The method of any one of E 1-17, wherein said determining of steps (b) and (c) is effected using strand-specific oligonucleotides.
E20. The method of E 19, further comprising sequencing said forward strand and said reverse strand.
E21. The method of any one of E 1-19, wherein steps (b) and (c) are carried out concomitantly in a single reaction vessel.
E22. The method of any one of E 1-19, wherein steps (b) and (c) are carried out in separate reaction vessels.
E23. The method of E 22, wherein step (b) and/or step (c) is effected using digital droplet PCR.
E24. The method of any one of E 1-22, wherein said double-stranded DNA is cell-free DNA.
E25. The method of any one of E 1-22, wherein said double-stranded DNA is cellular DNA.
E26. The method of E 25, wherein said method further comprises lysing the cells of said cellular DNA prior to said determining.
E27. The method of E 1, wherein the molecule is comprised in a body fluid sample.
E28. The method of E 27, wherein said body fluid is selected from the group consisting of blood, plasma, sperm, milk, urine, saliva and cerebral spinal fluid.
E29. The method of E 27, wherein said body fluid sample comprises DNA from a plurality of cell-types.
E30. The method of any one of E 27-29, wherein the sample is a blood sample.
E31. The method of any one of E 1-30, further comprising quantitating the amount of DNA of said cell or tissue origin.
E32. A kit for determining the methylation status of at least one methylation site of a double-stranded DNA molecule, the kit comprising:
   (i) a set of primers that are capable of amplifying the forward strand of the double-stranded DNA molecule and not the reverse strand of the double-stranded DNA molecule;
   (ii) a set of primers that are capable of amplifying the reverse strand of the double-stranded DNA molecule and not the forward strand of the double-stranded DNA molecule.
E33. The kit of E 32, further comprising bisulfite.
E34. The kit of E 32, wherein the double-stranded DNA molecule is differentially methylated in a first cell of interest with respect to a second cell which is non-identical to said first cell of interest.

## Claims

1. A kit for determining a methylation pattern of a double-stranded DNA target region, the kit comprising:
(a) a first pair of oligonucleotide primers configured, following bisulfite treatment of the double-stranded DNA target region, to amplify a first single-stranded DNA sequence derived from a forward strand of the double-stranded DNA target region and not to amplify a second single-stranded DNA sequence derived from a reverse strand of the double-stranded DNA target region; and
(b) a second pair of oligonucleotide primers configured, following the bisulfite treatment, to amplify the second single-stranded DNA sequence derived from the reverse strand and not to amplify the first single-stranded DNA sequence derived from the forward strand;
wherein the first and second single-stranded DNA sequences correspond to opposite strands of the same double-stranded DNA target region, and each comprises sequence positions corresponding to at least two CpG methylation sites in the target region; and
wherein the first and second pairs of oligonucleotide primers are configured to hybridize to respective primer-binding sites flanking the sequence positions corresponding to the CpG methylation sites such that amplification of the first and second single-stranded DNA sequences is independent of the methylation status of the CpG methylation sites.

2. The kit of claim 1, wherein the double-stranded DNA target region is present in a double-stranded DNA molecule no longer than 300 base pairs.

3. The kit of claim 2, wherein the double-stranded DNA molecule is no longer than 150 base pairs.

4. The kit of any preceding claim, wherein each of the first and second single-stranded DNA sequences comprises sequence positions corresponding to at least three CpG methylation sites in the target region.

5. The kit of any preceding claim, wherein the first and second pairs of oligonucleotide primers are configured for concomitant amplification of the first and second single-stranded DNA sequences in a single reaction vessel.

6. The kit of any preceding claim, further comprising:
(a) a first oligonucleotide probe configured to hybridize to a first amplification product generated using the first pair of oligonucleotide primers; and
(b) a second oligonucleotide probe configured to hybridize to a second amplification product generated using the second pair of oligonucleotide primers,
wherein the first and second oligonucleotide probes comprise distinguishable detectable labels.

7. The kit of claim 6, wherein each of the first and second oligonucleotide probes is configured to hybridize to its respective amplification product when at least one corresponding CpG methylation site was unmethylated in the double-stranded DNA target region before the bisulfite treatment.

8. The kit of any preceding claim, further comprising a bisulfite reagent.

9. The kit of any preceding claim, wherein the double-stranded DNA target region is differentially methylated in a first cell of interest relative to a second non-identical cell, and the second non-identical cell is a blood cell.

10. An in vitro method for determining a methylation pattern of a double-stranded DNA target region in a specimen, the method comprising:
(a) contacting double-stranded DNA from the specimen with bisulfite to generate:
(i) a first single-stranded DNA sequence derived from a forward strand of the double-stranded DNA target region; and
(ii) a second single-stranded DNA sequence derived from a reverse strand of the double-stranded DNA target region;
(b) amplifying the first single-stranded DNA sequence with a first pair of oligonucleotide primers that amplifies the first single-stranded DNA sequence and not the second single-stranded DNA sequence;
(c) amplifying the second single-stranded DNA sequence with a second pair of oligonucleotide primers that amplifies the second single-stranded DNA sequence and not the first single-stranded DNA sequence;
(d) determining a methylation status of at least two CpG methylation sites represented in each of a first amplification product produced in step (b) and a second amplification product produced in step (c); and
(e) determining the methylation pattern of the double-stranded DNA target region based on the methylation statuses determined for both the first and second amplification products.

11. The method of claim 10, wherein the amplifying of steps (b) and (c) is performed concomitantly in a single reaction vessel.

12. The method of claim 10, wherein the amplifying of steps (b) and (c) is performed in separate reaction vessels.

13. The method of any one of claims 10 to 12, wherein step (d) comprises sequencing each of the first and second amplification products.

14. The method of any one of claims 10 to 13, wherein:
(a) the double-stranded DNA comprises cell-free DNA obtained from a body-fluid specimen; and
(b) the body-fluid specimen comprises DNA from a plurality of cell types.

15. The method of claim 14, wherein:
(a) the double-stranded DNA target region is differentially methylated in a cell or tissue of interest relative to a non-identical cell or tissue; and
(b) the method further comprises quantitating DNA derived from the cell or tissue of interest based on the methylation pattern determined in step (e).
